(19) Europäisches Patentamt
European Patent Office
Office européen des brevets

(11) **EP 4 021 505 B1**

(12) **EUROPEAN PATENT SPECIFICATION**

(45) Date of publication and mention
of the grant of the patent:
**13.12.2023 Bulletin 2023/50**

(21) Application number: **20800353.3**

(22) Date of filing: **19.08.2020**

(51) International Patent Classification (IPC):
*A61K 41/00* (2020.01)    *A61P 31/04* (2006.01)
*A61P 31/12* (2006.01)    *C07K 16/28* (2006.01)
*A61K 31/43* (2006.01)    *A61K 31/496* (2006.01)
*A61K 31/5377* (2006.01)   *A61K 31/7036* (2006.01)
*A61K 39/395* (2006.01)   *A61K 39/00* (2006.01)
*C07K 16/24* (2006.01)

(52) Cooperative Patent Classification (CPC):
(C-Sets available)
**A61P 31/04; A61K 31/43; A61K 31/496;**
**A61K 31/5377; A61K 31/7036; A61K 39/3955;**
**A61K 41/0004; A61P 31/12; C07K 16/249;**
**C07K 16/2812; C07K 16/2833;** A61K 2039/505;
A61K 2039/507                    (Cont.)

(86) International application number:
**PCT/RU2020/050193**

(87) International publication number:
**WO 2021/040570 (04.03.2021 Gazette 2021/09)**

(54) **MEDICAMENT FOR TREATING INFECTIOUS DISEASES**

MEDIKAMENT ZUR BEHANDLUNG VON INFEKTIONSKRANKHEITEN

MÉDICAMENT POUR TRAITEMENT DE MALADIES INFECTIEUSES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Validation States:
**KH MD**

(30) Priority: **29.08.2019 RU 2019127185**
**29.08.2019 RU 2019127186**

(43) Date of publication of application:
**06.07.2022 Bulletin 2022/27**

(73) Proprietor: **Epshtein, Oleg Iliich**
**Moscow 127473 (RU)**

(72) Inventors:
• **PUSHKAR, Dmitry Yurievich**
**Moscow, 125009 (RU)**
• **EPSHTEIN, Oleg Iliich**
**Moscow, 127473 (RU)**

(74) Representative: **Leonard, Thomas Charles et al**
**Kilburn & Strode LLP**
**Lacon London**
**84 Theobalds Road**
**London WC1X 8NL (GB)**

(56) References cited:
**EP-A1- 1 295 606     EP-A1- 1 997 481**
**WO-A1-03/055517     WO-A2-2012/017328**

• **YANG JING ET AL: "Targeting**
**beta2-microglobulin for induction of tumor**
**apoptosis in human hematological**
**malignancies", CANCER CELL, CELL PRESS,**
**US, vol. 10, no. 4, 1 October 2006 (2006-10-01),**
**pages 295-307, XP009094069, ISSN: 1535-6108,**
**DOI: 10.1016/J.CCR.2006.08.025**

- NOBUO WATANABE ET AL: "A cell-based high-throughput screening assay system for inhibitor compounds of antigen presentation by HLA class II molecule", SCIENTIFIC REPORTS, vol. 7, no. 1, 28 July 2017 (2017-07-28), XP055770090, DOI: 10.1038/s41598-017-07080-4
- MR ROGER WYAND QC: "MR ROGER WYAND QC SITTING AS A DEPUTY HIGH COURT JUDGE Approved Judgment Epshtein v Comptroller-General Neutral Citation Number: [2016] EWHC 1511 (Ch): Case No: CH-2015-00022", Kluwer IP Law , 26 July 2016 (2016-07-26), XP002801893, Retrieved from the Internet: URL:http://patentblog.kluweriplaw.com/wp-content/uploads/sites/52/2016/07/here.pdf [retrieved on 2021-01-29]

(52) Cooperative Patent Classification (CPC): (Cont.)

C-Sets
**A61K 39/3955, A61K 2300/00**

**Description**

This invention relates to the field of pharmaceuticals, particularly to medicaments and methods of treating infectious diseases. In particular, the invention relates to products for treating infections of bacterial origin, for enhancing the therapeutic efficacy of antibiotics, including their effects against resistant bacteria and promotion of decreased effective doses of antibiotics, as well as a method of treating bacterial infections, a method of reducing bacterial resistance to antibiotic therapy, a method of reducing effective doses of antibiotics and a method of enhancing the therapeutic efficacy of antibiotics against antibiotic-specific resistant bacteria; the invention also relates to medicaments and methods for treating viral infections.

Infections can be classified as simple, i.e. infections induced by a single agent, and mixed infections, i.e. those induced by two or more agents. It is important to distinguish mixed infections from secondary infections (superinfections) arising from the current disease. Reinfection is defined by the reappearance of the disease caused by the same agent following the complete recovery and it is associated with lack of immunity.

Bacterial infections are diseases caused by bacteria. Bacterial infections are characterized by secretion of toxins by living and dead bacteria that lead to inflammation, intoxication and tissue damage. The diagnosis of bacterial infection requires prescription of antibacterial drugs. Bacteria that cause different diseases eventually develop resistance to antibiotics used in the treatment. This is a natural adaptive process that is called antimicrobial resistance. The development of a large number of resistant bacteria leads to a growing number of incurable infections, resulting in more increased risks of bacterial infections, higher medical costs associated with the treatment of bacterial infections, longer hospital stays and increased mortality [WHO. The evolving threat of antimicrobial resistance: Options for action. 2013].

In May 2015, the World Health Assembly endorsed a global action plan to tackle antimicrobial resistance, including antibiotic resistance. The goal of the global action plan is to ensure treatment and prevention of infectious diseases with effective and safer medicines [http://www.who.int/mediacentre/factsheets/antibiotic-resistance/ru/].

Acute upper respiratory tract infections (URIs) are the most common group of diseases in the world, comprising influenza, parainfluenza, infections caused by respiratory syncytial virus, rhinovirus and adenovirus, and other catarrhal inflammations of the upper respiratory tract. Viral transmission commonly occurs through self-inoculation of viruses onto nasal or conjunctival mucosae by hands after contact with an infected individual or contaminated surfaces. Another mode of transmission is airborne transmission that occurs due to inhalation of aerosols containing a virus or exposure of the mucosae to infective droplets when a person is in close contact with an infected individual. The absence of adequate treatment or ineffective therapy may lead to severe complications such as otitis media, sinusitis, tonsillitis, disseminated intravascular coagulation, etc.

The analysis of official statistical data from the World Health Organization (WHO) demonstrated that, at the beginning of the 21st century, infectious diseases were responsible for one quarter of all deaths in the world, while in developing countries the mortality from infectious diseases reached almost 50%. It can be stated that infectious diseases still remain one of the most important causes of mortality in population worldwide. The development of new therapeutic options against these diseases is a top-priority task in the medical and pharmaceutical industries.

Therefore, the treatment of viral and bacterial infections as well as ways to reduce antibiotic resistance and increase antibiotic susceptibility of bacteria have become challenges of current importance in the healthcare industry.

The prior art discloses drugs for treating infectious diseases, in particular antiviral agents, for example Arbidol, Amixin, etc [https://www.rlsnet.ru/tn_index_id_4196.htm, https://www.rlsnet.ru/tn_index_id_1526.htm]. Drugs of this class may have side effects and lack efficacy against certain viral diseases [Leneva I.A. et al. Characteristics of arbidol-resistant mutants of influenza virus: implications for the mechanism of anti-influenza action of arbidol. Antiviral Res 2009 81(2):132-40].

The prior art also discloses drugs for treating infectious diseases, in particular pharmaceutical products for treatment of bacterial infections such as Amoxicillin, Gentamycin, Tetracycline, Levomycetin, etc. [https://www.rlsnet.ru/tn_index_id_222.htm // https://www.rlsnet.ru/tn_index_id_876.htm // https://www.rlsnet.ru/mnn_index_id_82.htm // https://www.rlsnet.ru/tn_index_id_4699.htm]. Antibiotic drugs have two major problems: the increasing number of antibiotic-resistant microorganisms and a wide range of contraindications and side effects associated with their use [Belozertseva V.N. et al. Antibiotics: resistance and toxicity. New St. Petersburg Medical Records, 2017.-N 2. -P.59-61].

Thus, an inappropriate and uncontrolled use of antibiotics may be extremely harmful to human health. Apart from adverse events such as allergy, liver and kidney toxic sequelae, cardiovascular, blood and nervous system disorders, antibiotic treatment can also lead to the development of antibiotic-resistant microbes. Therefore, comprehensive efforts aimed at reducing antibiotic resistance of microorganisms and promoting wise use of antibiotics are of current importance.

A method to combat bacterial drug resistance is known and it implies the use of antibiotics in combination with bacterial enzyme inhibitors. For instance, clavulanic acid (or clavulanate), a beta-lactamase inhibitor, which has a chemical structure resembling beta-lactam antibiotics. Like other beta-lactams, clavulanic acid can bind to penicillin-binding proteins (PBPs) in gram-positive and gram-negative bacteria and contribute to lysis of the bacterial wall. Besides, clavulanic acid has its own antibacterial activity. Based on this, combination medicaments containing aminopenicillin and one of beta-lactamase inhibitors (amoxicillin/clavulanate) have been created. The disadvantage of this method is a limited spectrum of antagonistic effects of the used combinations of drugs, i.e. they are only effective against germs with beta-lactamase-mediated resistance. The side effects of clavulanic acid are dyspepsia, cholestatic jaundice, liver functional disorder, hepatitis, Clostridium difficile infection, candidiasis, and allergies (erythema multiforme, angiooedema, exfoliative dermatitis, allergic urticaria and anaphylactic shock) [https://www.kakprosto.ru/kak-899440-klavulanovaya-kislota-deystvie-i-svoystva-#ixzz5eej sFlpF] .

The prior art discloses a method of overcoming drug resistance of bacteria and fungi that enhances effective tackling of drug resistance of microorganisms to antimicrobials with different mechanisms of action due to combined effects of aniline dyes at low concentrations and antimicrobial agents [patent RU2363470C1, 10.08.2009]. It should be noted that aniline dyes are widely used in histology and exert antibacterial effects, while some of them demonstrate a carcinogenic action.

The prior art also discloses a pharmaceutical combination of active antibacterial and proteolytic enzymes for external and topical application. This composition comprises a base (for instance, lysoamidase) and at least one target supplement (antibiotic, synthetic antibacterial agent, including an agent reducing microbial resistance, antimycotic, anesthetic agent, or reparation stimulant). The invention ensures increased therapeutic activity of the pharmaceutical composition as compared to the known agents, including against strains that are resistant to individual components of the composition. It also improves the healing process, promotes faster wound cleansing and shorter time to convalescence [patent RU2655808C2, 29.05.2018]. It should be noted that enzymes are sensitive to different ambient factors (pH, temperature, pressure, etc.) and exposure to any external factors may result in denaturation and irreversible changes in the structure of an enzyme, followed by loss of its properties. For instance, it is recommended to store a lysoamidase enzyme at <_ +10 °C in dark, dry ambient, which presents additional challenges for its production, shipment and storage, as it may reduce its efficacy at any of these steps.

The prior art discloses a medicament containing an activated form of ultralow doses of monoclonal and polyclonal, immune or natural, antibodies to a major histocompatibility antigen (HLA complex) or to the HLA antigen complex and its associated peptide [patent RU2205025C1, 26.12.2001]. The immunotropic drug obtained in accordance with the invention is a new pharmaceutical agent, which is characterized by immunotropic activity, lack of side effects, environmental cleanliness and low cost. However, there are no data on the efficacy of this medicament in the treatment of bacterial and/or viral infections.

The prior art discloses a complex medicament to treat viral infections that contains an activated-potentiated form of antibodies to human interferon gamma and an activated-potentiated form of antibodies to CD4 receptor as active components [patent RU 2521392, 06.08.2010]. However, there are no data on the efficacy of this medicament in the treatment of bacterial or mixed infections.

Ergoferon is the closest analogue of the claimed medicament [https://www.rlsnet.ru/tn_index_id_46844.htm]. The pharmacological activity of Ergoferon includes antiviral and immunomodulatory effects; moreover, the medicament is also used in combination treatment of bacterial infections. Ergoferon contains a so-called activated-potentiated form of antibodies to human interferon gamma (IFN-$\gamma$), an activated-potentiated form of antibodies to CD4 of T cells (CD4) and an activated-potentiated form of antibodies to histamine (His).

An object of this invention is to create a new effective complex medicament that would demonstrate high antiviral, antibacterial and immunomodulatory activity and would also be effective against bacteria with resistance to one or more known antibacterial drugs (antibiotics), without any toxic or mutagenic effects on the patient organism. The invention is also aimed at the development of a medicament that could enhance the pharmacological efficacy of an antibiotic, in particular its efficacy against resistant bacteria, and reduce effective doses of an antibiotic.

The invention is as defined in the claims. In one embodiment, the medicament of the present invention is a technology-processed product resulting from multiple serial dilutions of the stock substances, namely antibodies to HLA-DRB1 and to $\beta$2-MG. In another embodiment, the medicament of the present invention is a technology-processed product resulting from multiple serial dilutions of the stock substances, namely a) HLA-DRB1, b) $\beta$2-MG, c) IFN-$\gamma$ and d) CD4. Both embodiments are effective for the treatment and prevention of bacterial infections caused by various types of pathogens.

One aspect of the invention is the claimed medicament for treating bacterial infections, which promotes enhanced efficacy of antibiotics, reduced bacterial resistance to antibiotic therapy and reduced effective doses of antibiotics and presents a technology-processed product resulting from multiple serial dilutions of the stock substances, i.e. antibodies to the $\beta$1 domain of the major histocompatibility complex class II molecule (HLA-DRB1) and antibodies to $\beta$2-microglobulin ($\beta$2-MG).

Another aspect of the invention is the claimed medicament with antibacterial and antiviral activity for treating infectious diseases, which presents a technology-processed product resulting from multiple serial dilutions of the stock substances: a) antibodies to the $\beta$1 domain of the major histocompatibility complex class II molecule (HLA-DRB1), b) antibodies to $\beta$2-microglobulin ($\beta$2-MG), c) antibodies to interferon gamma (IFN-$\gamma$) and d) antibodies to CD4.

The mentioned types of bacteria can be as follows, for example: spirochetes (for example, Treponema, Borrelia, Leptospira); gram-negative aerobic and microaerophilic, motile, curved and helical bacteria (for example, Campylobacter, Helicobacter, Spirillum); gram-negative aerobic and microaerophilic rods and cocci (for example, Achromobacter, Bordetella, Kingella, Neisseria); gram-negative facultative anaerobic rods (for example, Cedecea, Escherihia, Klebsiella, Plesiomona, Haemophilus, Streptobacillus (coli infections)); gram-negative anaerobic straight, curved and helical bacteria (for example, Anaerobiospirrilum, Bacteroides, Porphyromonas); gram-negative anaerobic cocci (for example, Veillonella); rickettsiae and chlamydiae (for example, Ehrlichia, Chlamydophila); gram-positive cocci (for example, Aerococcus, Staphylococcus, Streptococcus); gram-positive endospore-forming rods and cocci (for example, Bacillus, Clostridium); gram-positive non-spore-forming rods of regular shape (for example, Erysipelothrix, Listeria); gram-positive non-spore-forming rods of irregular shape (for example, Bifidobacterium, Corinebacterium, Rothia); mycobacteria (for example, Mycobacterium); actinomycetes (for example, Actinomadura, Nocardia, Streptomyces); mycoplasmas (for example, Mycoplasma, Ureaplasma), etc. [I.V. Smirnov. Pathogens of bacterial infections in humans. Clinical Microbiology and Antimicrobial Chemotherapy, No.2, Vol. 2, 2000, p.4-11].

The claimed medicament, which is a technology-processed product resulting from multiple serial dilutions of the stock substances of antibodies to HLA-DRB1 and to β2-MG, is also effective against bacteria that are resistant to one or more antibiotics.

Bacterial infections that can be managed by the claimed medicament can be grouped into classes based on both the type of a pathogenic bacterium and their mode of transmission:

Bacterial gastrointestinal/intestinal infections can be caused by shigella, staphylococci, cholera bacillus, typhoid bacillus or salmonella (salmonellosis, typhoid fever, dysentery, bacterial food poisoning, campilobacteriosis, enteritis, escherichiosis, colitis, food poisoning, gastritis, ulcer diseases, botulism food poisoning, etc.). The fecal-oral route of transmission is predominant for these infections.

Bacterial infections of the respiratory tract with airborne transmission can be caused by staphylococci, pneumococci, streptococci, Bordetella pertussis, meningococci, chlamydiae, mycobacteria, mycoplasma, etc. (sinusitis, rhinitis, acute tonsillitis, laryngitis, tracheitis, tonsillitis, epiglottitis, maxillary sinusitis, pneumonia, bronchitis, tuberculosis, etc.).

Bacterial infections of the genitourinary system, such as bacterial vaginosis (gardnerellosis), chlamydia, cystitis, dysbacteriosis, pyelonephritis, glomerulonephritis, urethritis, bacterial balanitis, prostatitis, bacterial cystitis, bacterial prostatitis, seminal vesiculitis, etc.;

Skin and soft tissue bacterial infections caused by, for example, staphylococci and streptococci, are transmitted by skin contact (erysipelas, impetigo, cellulitis, phlegmon, furunculosis, hidradenitis, etc.).

Blood infections caused by, for instance, rickettsiae, yersinia, and coccobacilli have a transmissive route (rabbit fever, plague, spotted fever, trench fever, etc.).

Infections of the lymphatic system, such as lymphatic gland inflammation and lymphangitis, can be caused by streptococcus or staphylococcus;

Infections of the nervous system, for instance, meningitis, encephalitis, myelitis and other infections, caused by meningococci, pneumococci, Haemophilus influenzae type b, Borrelia, mycoplasma, Candida fungi, etc., can be transmitted by the airborne route as well as through the lymphatic system;

Bone and joint infections can be caused by bacterial agents of Lyme disease, staphylococci, streptococci, mycobacteria and gonococci and transmitted via the bloodstream, through direct contact, for example, open wound or surgical procedures, etc., and through infections in the nearest body structures (septic arthritis, osteomyelitis, Reiter's syndrome, etc.);

Ear infection (mastoid and sinus infections) can be caused by streptococci and staphylococci, Candida fungi, Haemophilus influenza, etc. The routes of transmission are through the Eustachian tube, from the external auditory canal, or by blood (labyrinthitis, sensorineural hearing loss, mastoiditis, etc.).

The pharmaceutical composition of the present invention is a combination of active ingredients for the treatment of bacterial infections, i.e. it is a medicament which presents a technology-processed product resulting from multiple serial dilutions of the stock substances of antibodies to HLA-DRB1 and antibodies to β2-MG and the corresponding antibiotic, when both being co-administered to a patient. A co-administration of the medicament and the corresponding antibiotic can be carried out either by a simultaneous intake of the drugs as two independent products (as individual dosage forms) at the same time or successively within the required time intervals, in particular when these intervals allow a combination of the products to exert improved effects. The method of choice is determined based on experience and depends on the disease and the antibiotic used.

The claimed medicament, together with the known antibiotic, can also compose either a fixed or a non-fixed pharmaceutical combination used for the treatment of bacterial infections. The term "fixed combination" indicates that the active ingredients, i.e. the claimed medicament and a partner drug (antibiotic), are co-administered simultaneously to a patient as a single pharmaceutical form or dose. The terms "non-fixed combination" or "set of components" indicates that the active ingredients, i.e. the claimed medicament and a partner drug (antibiotic), are co-administered to a patient as separate components either simultaneously or successively without any specific time intervals in the cases where such administration ensures effective levels of the two active ingredients in the body. The claimed combination of the medicament and antibiotic can be administered either simultaneously or successively for the treatment of bacterial infections, with the antibiotic being administered at the known effective dose.

The claimed medicament (antibodies to HLA-DRB1 and antibodies to β2-MG) is used at the effective dose selected by experiment, while an antibiotic should be taken in compliance with the patient information leaflet or as prescribed by the doctor. The antibiotic can be selected from the following groups, for example: beta-lactam antibiotics (penicillins (amoxicillin, methicillin, oxacillin, benzylpenicillin, etc.); cephalosporins (cephalexin, cefazolin, ceftriaxone, cefepime, etc.); carbapenems (meropenem, doripenem, biapenem, etc.)); macrolides (ketolides, erythromycin, roxithromycin, clarithromycin, etc.); tetracyclines (tetracycline, oxytetracycline, doxycycline, metacycline, etc.); aminoglycosides (streptomycin, kanamycin, amikacin, gentamycin, etc.); chloromycetins; oxazolidinones; glycopeptides (targocid, daptomycin, vancomycin, etc.); lincosamides (dalacin C); fluoroquinolones (levofloxacin, gemifloxacin, sparfloxacin, etc.); antifungal antibiotics (nystatin, amphotericin B, etc.); antituberculosis antibiotics (phthivazide, methazide, saluzide, etc.); antileprotic drugs (solusulfon, diuciphon, etc.); antineoplastic agents (doxorubicin, rubomycin, carminomycin, etc.); and other antibiotics (phosphomycin, fusidin, rifampicin, etc.) [https://ru.wikipedia.org/wiki/Антибиотики].

The effective dose (ED) or effective concentration (EC) is defined as a dose or concentration of the medicament that produces a biological response. The term "effective dose" is used with regard to in vivo measurements, whereas the term "effective concentration" is used with regard to in vitro measurements.

The current invention is intended to enhance the pharmacological activity of antibiotic, in particular its effects against resistant bacteria, via a simultaneous or successive co-administration of the corresponding antibiotic and the claimed medicament, which presents a technology-processed product resulting from multiple serial dilutions of the stock substances of antibodies to HLA-DRB 1 and to β2-MG. Thus, a co-administration of the claimed medicament and antibiotics promotes reduction of antibiotic doses.

The medicament of the present invention, which is a technology-processed product resulting from multiple serial dilutions of the stock substances, namely a) HLA-DRB1, b) β2-MG, c) IFN-γ and d) CD4, can be used in the treatment of viral infections. The mentioned viral infections can be caused by the following viruses: double-stranded DNA viruses (for example, Herpesvirales, Adenoviridae, Papillomavirida, Polyomaviridae.); single-stranded DNA viruses (for example, Circoviridae, Parvoviridae); viruses with RNA capable of replication (for example, Reoviridae, Birnaviridae); positive-sense single-stranded RNA viruses (for example, Nidovirales, Picornavirales, Tymovirales, Astroviridae, Caliciviridae, Flaviviridae, Togaviridae, Virgaviridae); negative-sense single-stranded RNA viruses (for example, Bunyavirales, Mononegavirales, Arenaviridae, Ophioviridae, Orthomyxoviridae, Deltavirus); positive-sense single-stranded RNA viruses that replicate through a DNA intermediate (for example, Retroviridae); and double-stranded DNA viruses that replicate through a single-stranded RNA intermediate (for example. Caulimoviridae, Hepadnaviridae) [https://ru.wikipe-dia.org/wiki/Классификация_вирусов_по_Балтимору].

Moreover, the present medicament, which is a technology-processed product resulting from multiple serial dilutions of the stock substances, namely a) HLA-DRB1, b) β2-MG, c) IFN-γ and d) CD4, is effective against mixed infections that arise due to concurrent exposure to two or more different pathogens, both viruses and bacteria, and secondary infections (superinfections) that occur following an infection from another pathogen.

The technical result of the present invention is to broaden the range of medicaments with complex antiviral and antibacterial effects and to create a broad-spectrum antibacterial agent that would be effective against bacteria that are resistant to one or more antibiotics and would demonstrate low toxicity or mutagenicity, thus reducing the burden on a patient's organism during antibacterial therapy. Moreover, the claimed medicament enhances the pharmacological efficacy of an antibiotic, including efficacy against resistant bacteria, and reduces its effective dose, minimizing side effects during and after antibiotic treatment.

The technology-processed products resulting from multiple serial dilutions of the stock substances of antibodies to HLA-DRB1, antibodies to β2-MG and antibodies to IFN-γ, antibodies to CD4 are aqueous or aqueous-alcoholic solutions of antibodies (or a combination of these solutions) obtained by serial dilutions (potentization) of the antibody matrix solution, with each dilution subjected to a succussion process.

In the previous works [refer to RU2205025C1, 26.12.2001; RU2500422C2 06.08.2010, etc.], the applicant used the terms 'activated form of antibodies', 'activated-potentiated form of antibodies', 'ultrahigh dilutions' and 'ultralow doses of antibodies' to describe the technology-processed products resulting from multiple serial dilutions of antibody stock substances. The applicant also highlights that the term 'released-active form' and the terms 'activated form of antibodies', 'activated-potentiated form of antibodies', 'ultralow doses of antibodies', 'ultrahigh dilutions' and 'active dilutions' can be used interchangeably [Epstein O.I. Released-activity (contemporary view on homeopathy and non-homeopathy). Moscow: RAMS Publishing House, 2017. 48 p. // Epstein O.I. Ultralow doses (history of one study). Moscow: RAMS Publishing House, 2008. 336 p.].

In this invention, an antibody dilution is defined as any dilution of the antibody matrix solution, including decimal, centesimal and millesimal dilutions, from C2 and onwards (i.e. from the matrix solution diluted $100^2$ times) as well as any combinations thereof (for example, C2+D34+M45, D20+C4, etc.) and any dilution ratios (for instance, 1:1:2, 2:3, etc.).

The claimed medicament is prepared with the use of natural, monoclonal or, mainly, polyclonal antibodies as a stock substance that can be obtained according to the known technologies and methods as described in: Myagkova M.A., Morozova V.S. Immunochemical properties of natural antibodies to physiologically active compounds, Fundamental Research No. 11, 2014, p.1066-1070 and Immunological methods edited by G. Frimel, Moscow, Meditsina, 1987, p.9-33; or, for example, in the article by Laffly E., Sodoyer R. Hum. Antibodies. Monoclonal and recombinant antibodies, 30 years after. - 2005 - Vol.14. - N 1-2. P.33-55, and that have antigen specificity to the corresponding antigens, i.e. HLA-DRB1, $\beta$2-MG, IFN-$\gamma$ and CD4.

Natural antibodies are defined as immunoglobulins secreted by the body in limited amounts in the absence of exogenous antigen stimulation, that is why these antibodies are also present in the circulation of healthy humans. Natural autoantibodies appear to be primarily polyreactive antibodies of low affinity to a certain set of autoantigens.

Monoclonal antibodies are heterogeneous antibodies that recognize a specific antigenic epitope and are produced by a single B cell clone [Lipman NS1, Jackson LR, Trudel LJ, Weis-Garcia F. Monoclonal versus polyclonal antibodies: distinguishing characteristics, applications, and information resources. ILAR J. 2005;46(3):258-68. // IHC Staining Methods. Fifth edition. Preface Chapter 1. Thomas Boenisch. Antibodies. p:1-9]. Monoclonal antibodies can be produced using the hybridoma technology. This process starts with immunization based on the pre-developed techniques used for production of polyclonal antisera. The further steps of the process are performed to grow hybrid cells that produce antibody clones of a single specificity. Their individual isolation is carried out using the same methods as for polyclonal antisera.

Polyclonal antibodies are presented by a collection of immunoglobulins that recognize multiple epitopes of a specific antigen and are secreted by different B cell lines within the body [Lipman NS1, Jackson LR, Trudel LJ, Weis-Garcia F. Monoclonal versus polyclonal antibodies: distinguishing characteristics, applications, and information resources. ILAR J. 2005;46(3):258-68. // IHC Staining Methods. Fifth edition. Preface Chapter 1. Thomas Boenisch. Antibodies. p:1-9]. Polyclonal antibodies can be produced by active immunization of animals. For immunization, animals are given injections of a suitable compound required by the invention, i.e. antigen, according to the developed injection protocol. As a result, this procedure gives monospecific antisera with a high concentration of antibodies that are used in the method of multiple serial dilutions to obtain the finished product. Affinity purification of antibodies present in the antiserum can be performed, for example, using the fractional precipitation method or ion exchange chromatography, if necessary.

Given the above, the term 'antibodies' refers to immunoglobulins of any origin (natural, polyclonal or monoclonal antibodies) that specifically bind to a target molecule (antigen). Specificity, i.e. the capability of antibodies to recognize and interact with a specific type of antigenic epitopes, is a central characteristic of an antibody that defines the whole range of its effects [Boyd WC. Fundamentals of immunology. Fundam. Immunol. 1946; Langman RE. The specificity of immunological reactions. Mol. Immunol. 2000; 37: 555-561]. Specificity does not depend on the nature of antibodies or the method of their production: natural, polyclonal or monoclonal antibodies of identical specificity will affect the same target [A. Roitt et al., Essential Immunology. Translated from English. - Moscow: Mir, 2000, p.149-161, 527-544]; and therefore they can be used to produce the claimed medicament.

In one embodiment, the claimed medicament can be prepared using polyclonal antibodies as a matrix (stock) solution at a concentration of $0.5 \div 5.0$ mg/ml for technology processing conducted by multiple serial dilutions of antibody stock substances to obtain the finished product.

Technology processing, i.e. a method of multiple serial dilutions of antibody stock substances, is a systematic reduction of the concentration through serial dilutions of 1 part of said matrix solution in 9 parts (for decimal dilutions, D) or 99 parts (for centesimal dilutions, C) or 999 parts (for millesimal dilutions, M) of a neutral solvent in combination with external mechanical action (for instance, shaking) applied to each of the dilutions obtained, and when a new container is used for each successive dilution [refer to W. Shwabe "Homeopathic medicines", Moscow, 1967, p.14-29].

A seemingly simple procedure of repeated and successive reduction in the concentration of substances turns out to be a complicated technology that delivers products with unique properties. Historically, products of the potentization technology have been called 'small doses', 'potentiated preparations' or 'high dilutions'.

The finished product can be produced in different dosage forms [OFS.1.6.2.001.18, the State Pharmacopoeia of the Russian Federation, ed. XIV] and can contain pharmaceutically acceptable excipients.

For instance, the claimed medicament can be produced as a solid dosage form containing the required (effective) quantity of a pharmaceutically acceptable excipient, which is presented by a neutral carrier (for example, lactose) impregnated with a mixture of technology-processed products resulting from multiple serial dilutions of the antibody stock substances, such as antibodies to HLA-DRB1, $\beta$2-MG, IFN-$\gamma$, CD4, and pharmaceutically acceptable excipients, such as hypromellose, maltitol, glycerol, potassium sorbate, anhydrous citric acid, isomalt, silicon dioxide, sodium cyclamate, sodium saccharin, microcrystalline cellulose, magnesium stearate, etc.

The finished product of this invention is a medicament, which is a technology-processed product resulting from multiple serial dilutions of the stock substances: a) antibodies to the β1 domain of the major histocompatibility complex class II molecule (HLA-DRB1) and b) antibodies to β2-microglobulin (β2-MG) or a) antibodies to the β1 domain of the major histocompatibility complex class II molecule (HLA-DRB1), b) antibodies to β2-microglobulin (β2-MG), c) antibodies to interferon gamma (IFN-γ) and d) antibodies to CD4. The dilutions of the stock substances used may vary from decimal (D) to millesimal (M) dilutions, having different dilution ratios. The optimal dilutions and ratios of the initial components are determined in compliance with the known principles of pharmacodynamics and pharmacokinetics, in particular a dose-response curve.

The solid oral dosage form of the claimed medicament is prepared on the fluid bed system (for example, Hüttlin Pilotlab by Hüttlin GmbH) by spraying the previously prepared aqueous or aqueous-alcoholic solution (the concentration is selected experimentally, OFS.1.6.2.0010.18, State Pharmacopoeia) of the technology-processed products resulting from multiple serial dilutions of antibody stock substances onto a fluidized bed of neutral carrier granules, i.e. lactose (milk sugar), that are simultaneously dried by an upward current of heated air at <_ 40°C. The tablet blend obtained is mixed uniformly and then compacted by dry direct compression method (for example, in the XL 400 tablet press by Korsch) [WO2007105981(A1), 20.09.2007]. The compression process yields 300 mg tablets impregnated with an aqueous or aqueous-alcoholic solution of the technology-processed products resulting from multiple serial dilutions of the stock substances, i.e. antibodies to HLA-DRB1, antibodies to β2-microglobulin, antibodies to interferon gamma and/ or antibodies to CD4.

The figures below, together with the drawings attached, are offered to illustrate the present invention:

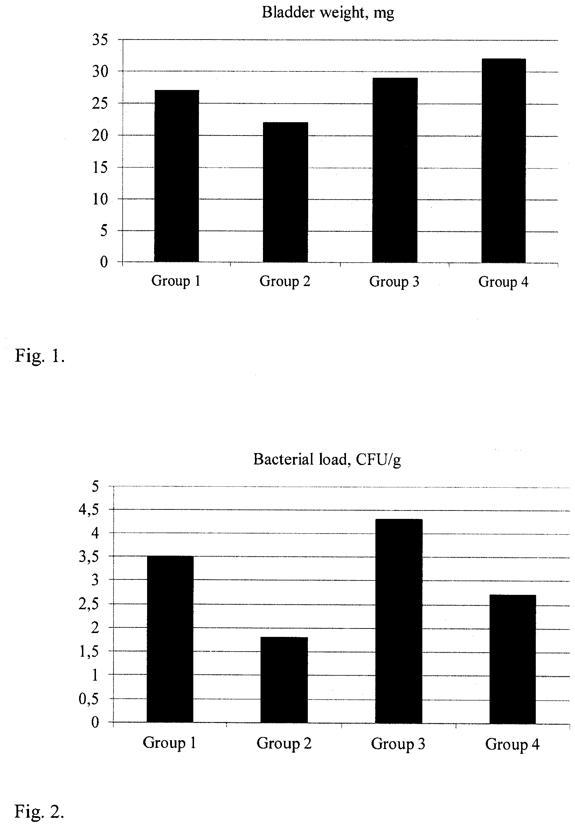

Fig.1. Bladder weight 7 days post infection.

Fig.2. Bladder bacterial load in mice 7 days post infection.

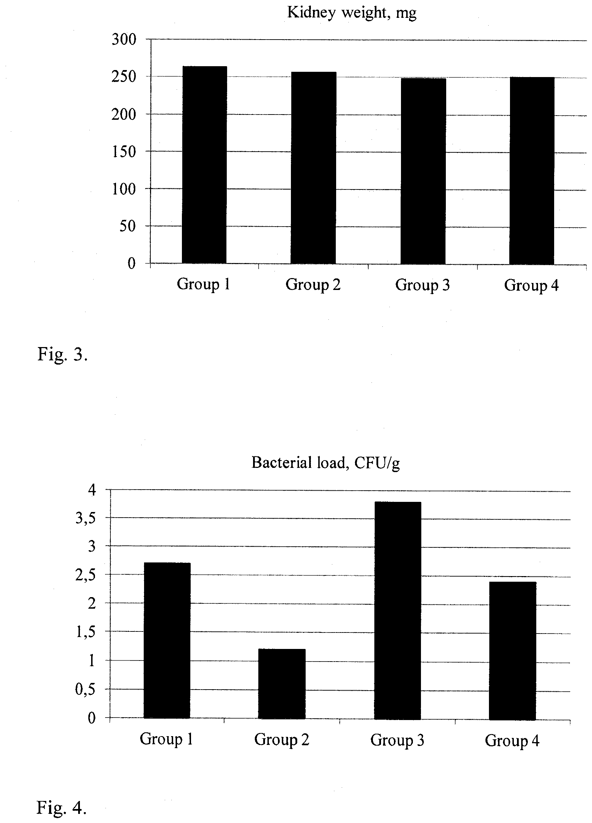

Fig.3. Kidney weight on day 7 post infection.

Fig.4. Kidney bacterial load in mice 7 days post infection.

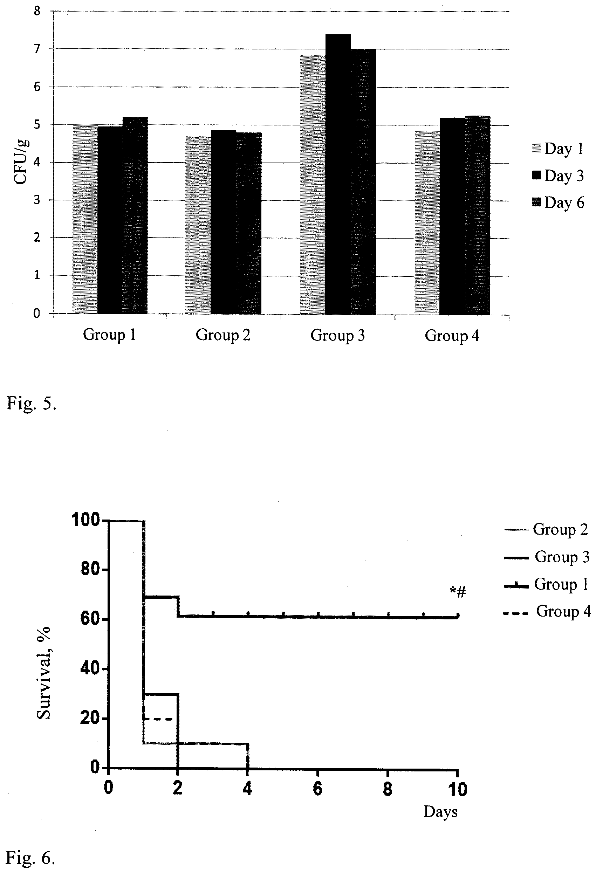

Fig.5. Urine bacterial load 1, 3 and 6 days post infection.

Fig. 6. Mice survival (%) after Escherichia coli infection with a dose of 2LD100 (point 0 on the horizontal axis). Note: * - the difference from the control group is statistically significant, $p < 0.05$; # - the difference from the enrofloxacin group is statistically significant, $p < 0.05$.

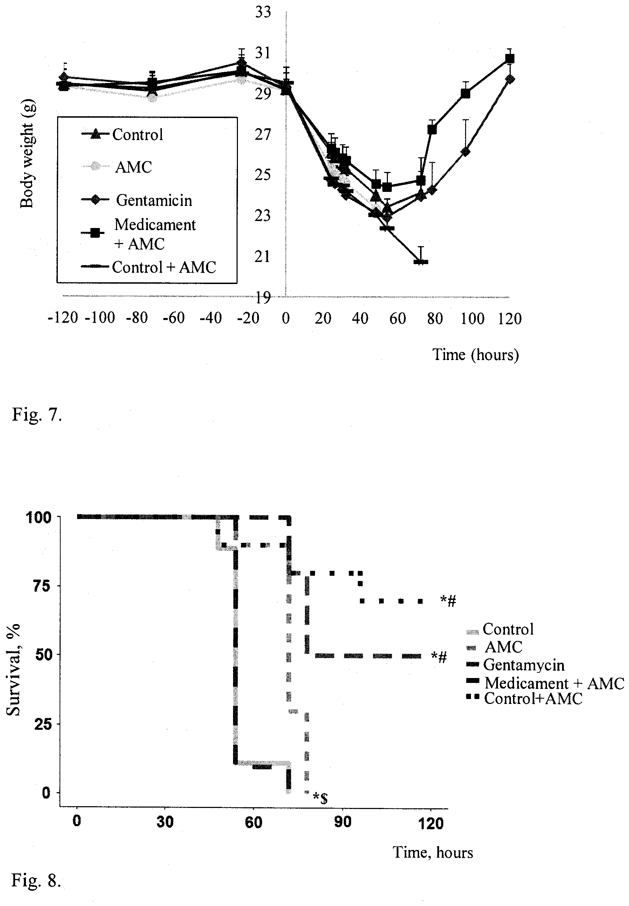

Fig.7. Mice body weight (M±m) as a marker of the antibacterial activity of a combination of the claimed medicament and AMC after infection (point 0 on the horizontal axis) with AMC-resistant Klebsiella pneumoniae BAA-1705 strain at a dose of 106 CFU/mouse.

Fig.8. Mice survival (%) as a marker of the antibacterial activity of a combination of the claimed medicament and AMC after infection (point 0 on the horizontal axis) with AMC-resistant Klebsiella pneumoniae BAA-1705 strain at a dose of 106 CFU/mouse. Note: * - the difference from the control group is statistically significant, $p < 0.05$; # - the difference from the Control+AMC group is statistically significant, $p < 0.05$; $ - the difference from the AMC group is statistically significant, $p < 0.05$.

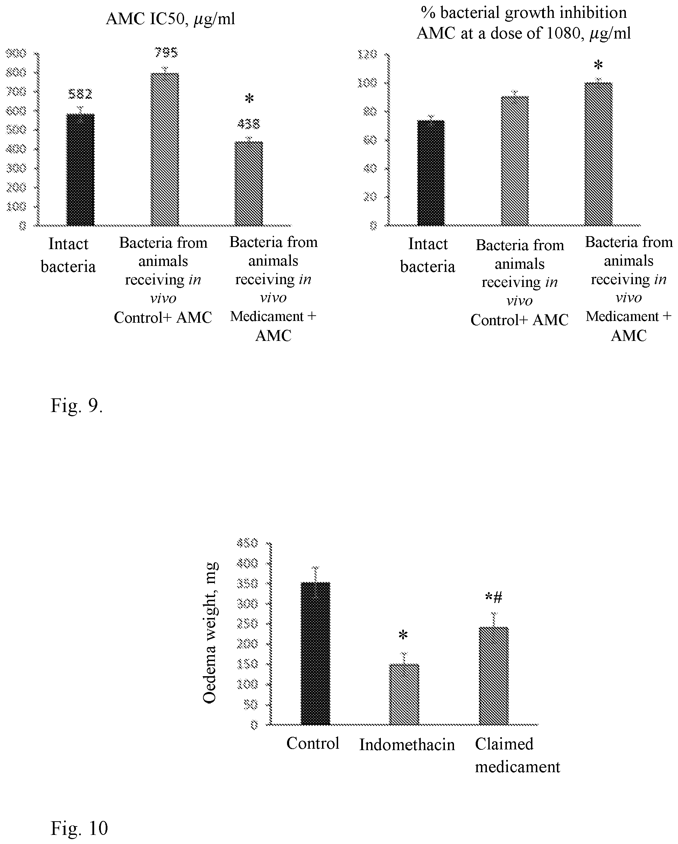

Fig.9. The AMC 50% inhibitory concentration (IC50, M±m) (Klebsiella pneumoniae BAA-1705 strain, $5 \times 104$ CFU/well) and bacterial sensitivity (inhibition %) to the AMC 100% inhibitory concentration for intact bacteria and for bacteria isolated from animals that received the test items in vivo. Note: * - the difference from the 'intact bacteria' value is statistically significant.

Fig.10 Oedema weight (M±m) as a marker of the anti-inflammatory activity of the claimed medicament in a model of carrageenan induced paw oedema in rats. Note: * - the difference from the control group is statistically significant, $p < 0.05$; # - the difference from the indomethacin group is statistically significant, $p < 0.05$.

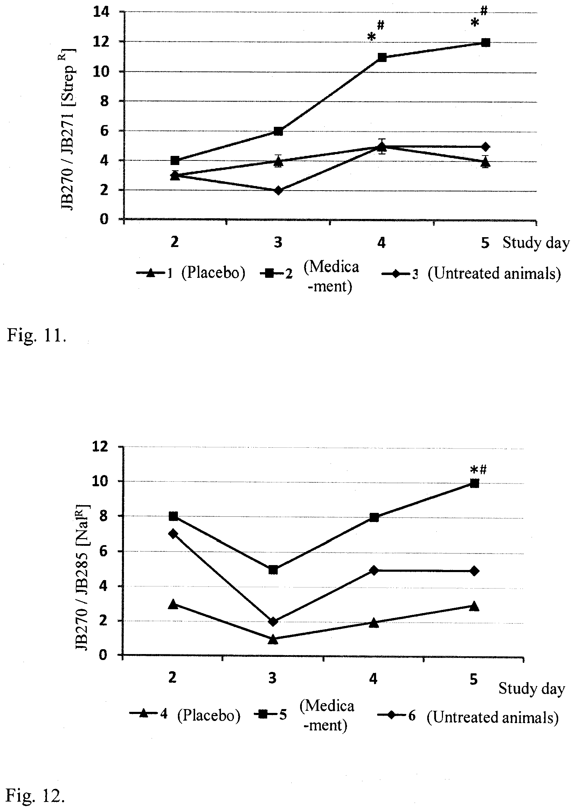

Fig.11. Comparison between the number of CFU of the streptomycin-sensitive Salmonella enterica strain (JB270) and that of the streptomycin-resistant Salmonella enterica strain (JB271 [StrepR]) per g of spleen in mice on days 2-4 after infection with JB270 + JB271 [StrepR] (1:1 at a dose of 5.0 log10 CFU/mouse). The data are presented as M±SE. * $p < 0.05$ vs the values obtained in the same group on day 2 and 3 of the study; # $p < 0.05$ vs the values obtained in groups 1 and 3 on days 2, 3, 4 and 5 of the study (day 1, 2, 3 and 4 after infection, respectively).

Fig.12. Comparison between the number of CFU of the nalidixic acid-sensitive Salmonella enterica strain (JB270) to that of the resistant Salmonella enterica strain (JB285 [NalR]) per g of spleen in mice on days 2-4 after infection with JB270 + JB285 [NalR] (1:1 at a dose of 5.0 log10 CFU/mouse). The data are presented as M±SE. * $p < 0.05$ vs the values obtained in the same group on day 3 of the study; # $p < 0.05$ vs the values obtained in groups 4 and 6 on days 3, 4 and 5 of the study (day 2, 3 and 4 after infection, respectively), as well as in group 4 on day 2 of the study.

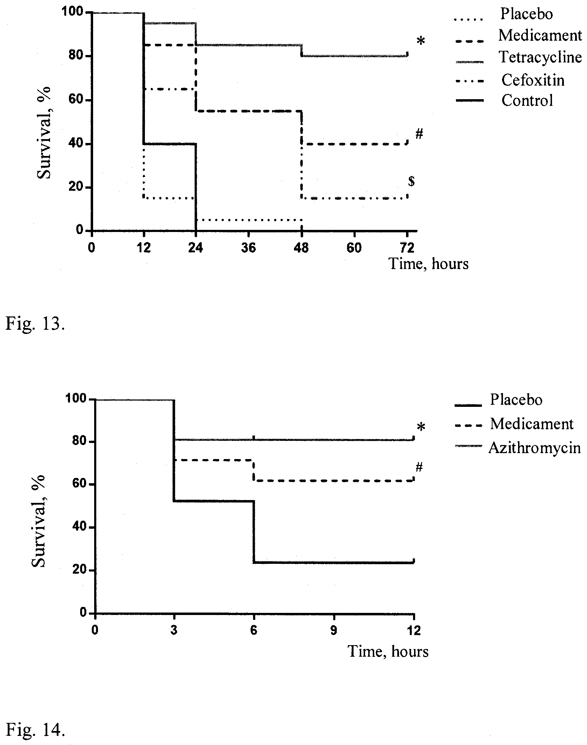

Fig. 13 Mice survival (%) as a marker of the antibacterial activity of the claimed medicament after infection (point 0 on the horizontal axis) with Clostridium perfringens (ATCC 13124) by intraperitoneal route (10^10 CFU/mouse). * $p < 0.05$ vs the values obtained in groups 1, 2, 4 and 5; # $p < 0.05$ vs the values obtained in groups 1, 4 and 5; $ $p < 0.05$ vs the values obtained in groups 1 and 5.

Fig. 14. Mice survival (%) as a marker of the antibacterial activity of the claimed medicament in the treatment of animals infected with (point 0 on the horizontal axis) Chlamydophila pneumonia (CM1) by intratracheal route (1.5*10^6

CFU/mouse). * p < 0.05 vs the values obtained in groups 1 and 2; # p < 0.05 vs the values obtained in group 1.

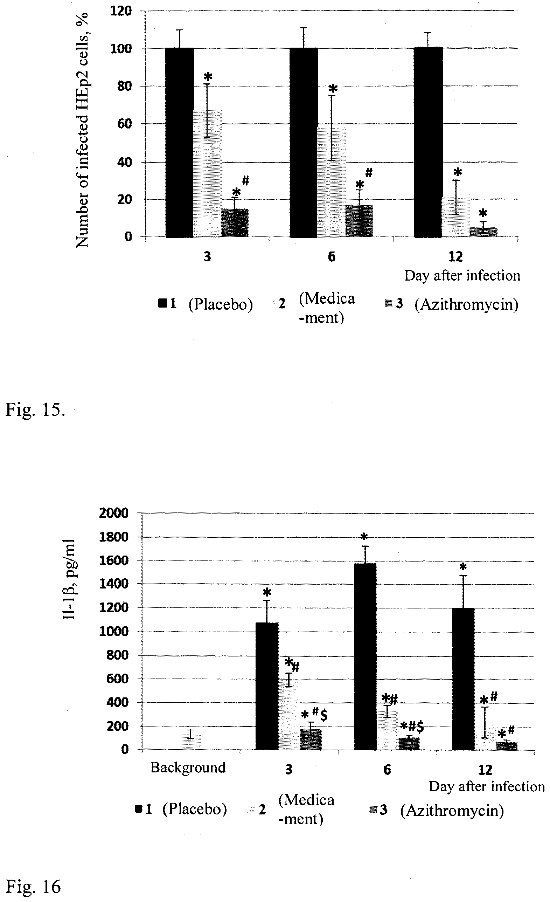

Fig. 15. The number of HEp2 cells infected with Chlamydophila pneumonia (CM1 strain) following incubation for 72 hours with lung tissue homogenate obtained from mice on days 3, 6 and 12 after infection with identical bacteria by intratracheal route ($1.5*10^6$ CFU/mouse) that received placebo, the claimed medicament or azithromycin. The data are presented as M±SE, as % of the control - 1 (Placebo). * p < 0.05 vs the values obtained in group 1 (Placebo) on the same day of the study; # p < 0.05 vs the values obtained in group 2 (Medicament) on the same day of the study.

Fig. 16. The interleukin-1β concentration in lung tissue of mice as a marker of the immunotropic activity of the claimed medicament in the treatment of animals infected with (point 0 on the horizontal axis) Chlamydophila pneumonia (CM1) by intratracheal route ($1.5*10^6$ CFU/mouse). The data are presented as M±SE. * p < 0.05 vs the background values (intact); # p < 0.05 vs the values obtained in Group 1 (Placebo); $ p < 0.05 vs the values obtained in group 2 (Medicament). Comparisons between the groups were carried out within a single timepoint (study day).

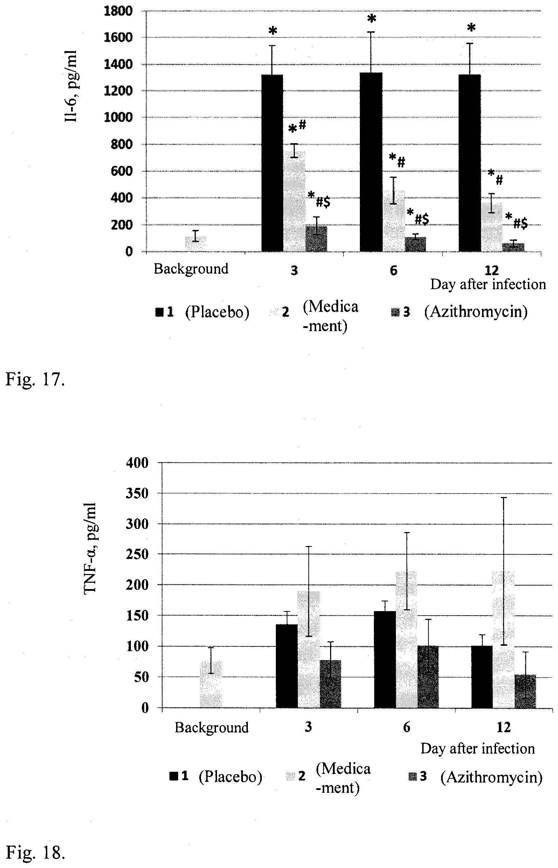

Fig. 17. The interleukin-6 concentration in lung tissue of mice as a marker of the immunotropic activity of the claimed medicament in the treatment of animals infected with (point 0 on the horizontal axis) Chlamydophila pneumonia (CM1) by intratracheal route ($1.5*10^6$ CFU/mouse). The data are presented as M±SE. * p < 0.05 vs the background values (intact); # p < 0.05 vs the values obtained in group 1 (Placebo); $ p < 0.05 vs the values obtained in group 2 (Medicament). Comparisons between the groups were carried out within a single timepoint (study day).

Fig. 18. Treatment effects on the TNF-α concentration in lung tissue of mice infected with (point 0 on the horizontal axis) Chlamydophila pneumonia (CM1) by intratracheal route ($1,5*10^6$ CFU/mouse). The data are presented as M±SE.

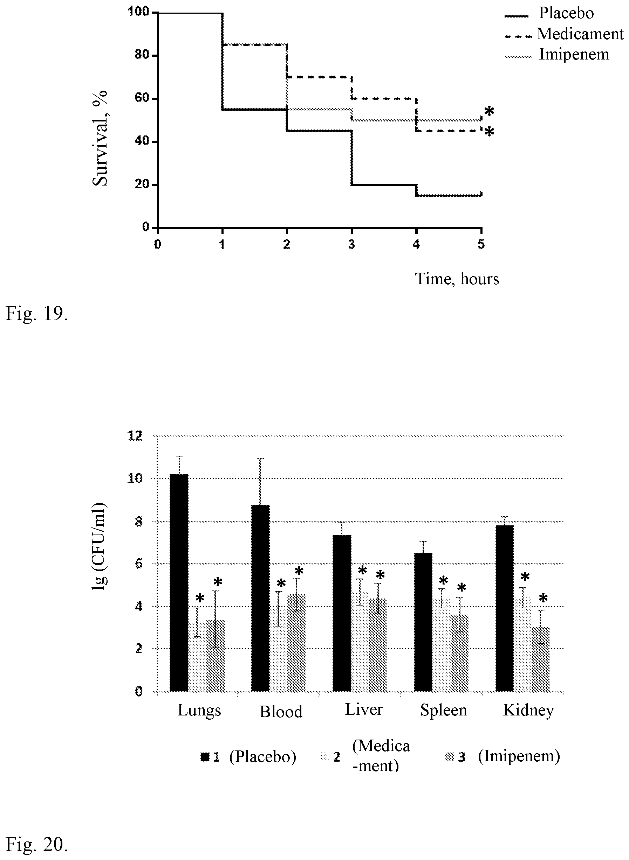

Fig. 19. Mice survival (%) as a marker of the antibacterial activity of the claimed medicament in the treatment of neutropenic animals infected with (point 0 on the horizontal axis) Klebsiella pneumoniae 8637 strain by intranasal route ($2*10^7$ CFU/mouse). * p < 0.05 vs the values obtained in the control (placebo).

Fig. 20. Bacteria load in the blood and organs of mice infected with (point 0 on the horizontal axis) Klebsiella pneumoniae 8637 strain by intranasal route ($2*10^7$ CFU/mouse). * p < 0.05 vs the values obtained in the control (placebo).

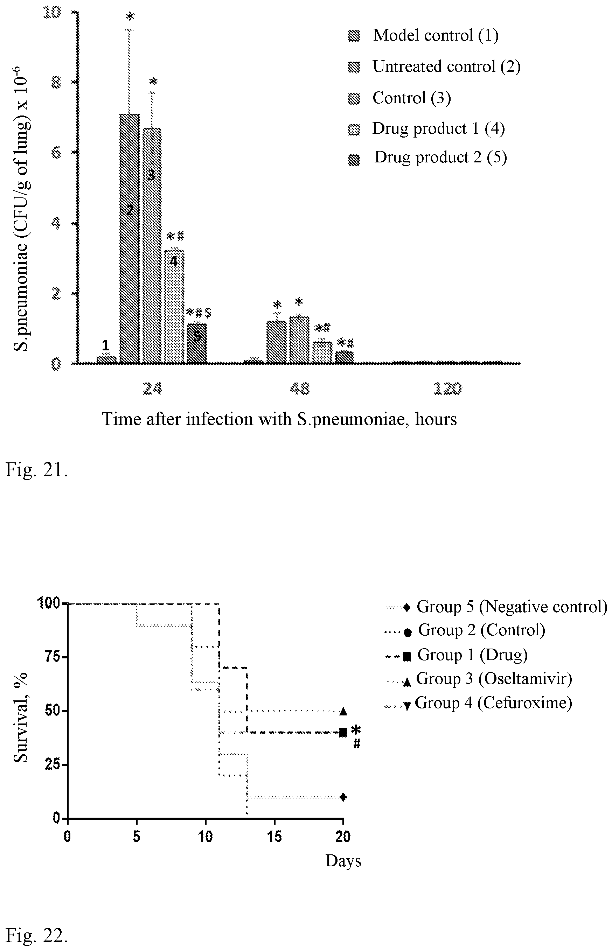

Fig. 21. Effects of Medicament 1 and Medicament 2 on the CFU number of S. pneumoniae in mice lungs after sequential infection with A/New Jersey/8/76 (H1N1) virus and S. pneumoniae. Note: * - the difference from the "Model control" group is statistically significant, p<0.05; # - the difference from the "Untreated control" and "Control" groups is statistically significant, p<0.05; $ - the difference from the "Medicament 1" group is statistically significant, p<0.05.

Fig.22. Mice survival (%) after infection with (point 0 on the horizontal axis) H1N1/A California 04/2009 influenza virus and Staphylococcus aureus (1986 strain). Note: * - the difference from the control group is statistically significant, p<0.05; # - the difference from the negative control group is statistically significant, p<0.05.

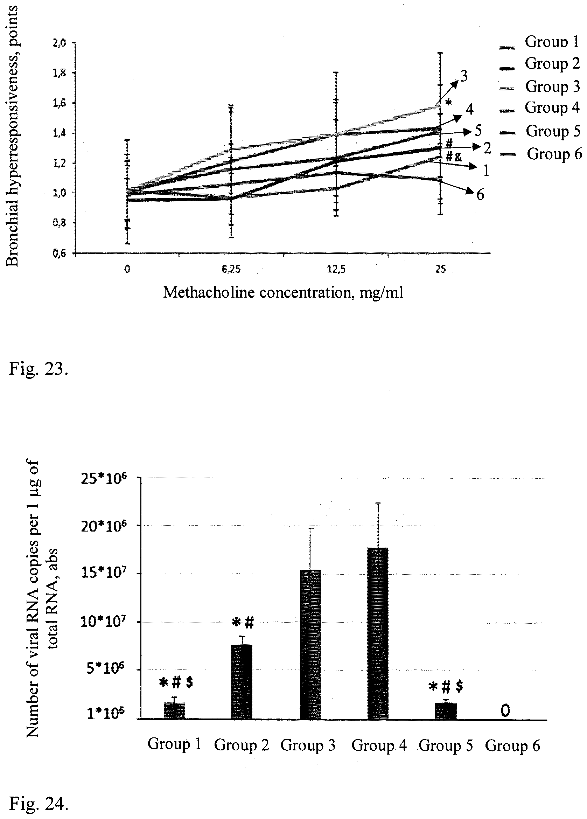

Fig. 23. Changes in the relative hyperresponsiveness of the bronchi in mice infected with RSV (A2 strain). Note: in the diagram, bronchial hyperresponsiveness data per group at the point "0 mg/ml methacholine" (baseline) were taken as 1. The values obtained at other points were normalized to the baseline of the corresponding group. The data are presented as normalized M±CI 95%. * - the difference from the intact group (Group 6) is statistically significant, p<0.05; # - the difference from the control group (Group 3) is statistically significant, p<0.05; & - the difference from the RSV group (Group 4) is statistically significant, p<0.05.

Fig.24. The number of viral RNA copies (M±m) in the lungs of mice infected with RSV (A2). Note: * - the difference from the control (Group 3) and RSV (Group 4) groups is statistically significant, p<0.05; # - the difference from the Group 6 is statistically significant, p<0.05, $ - the difference from the Group 2 is statistically significant, p<0.05

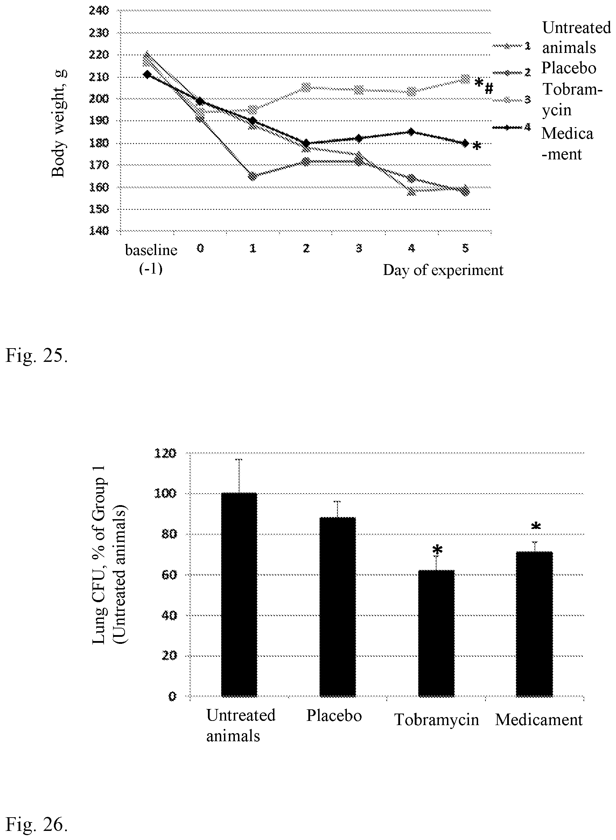

Fig. 25. The body weights of rats infected with Pseudomonas aeruginosa (RP73) by intratracheal route at a dose of $6.5*10^7$ CFU/ml that received placebo, the claimed medicament or tobramycin as therapeutic and preventive therapy. The data are presented as M±SE. * p < 0.05 vs the values obtained in Group 1 (Untreated animals) and group 2 (Placebo); # p < 0.05 vs the values obtained in Group 4 (Medicament).

Fig. 26. Bacterial load in rat lungs on day 5 after intratracheal infection with Pseudomonas aeruginosa (RP73) at a dose of $6.5*10^7$ CFU/ml and administration of placebo, the claimed medicament or tobramycin as therapeutic and preventive therapy. The data are presented as % of the values obtained in Group 1 (Untreated animals), M±SE. * p < 0.05 vs the values obtained in Group 1 (Untreated animals) and in group 2 (Placebo).

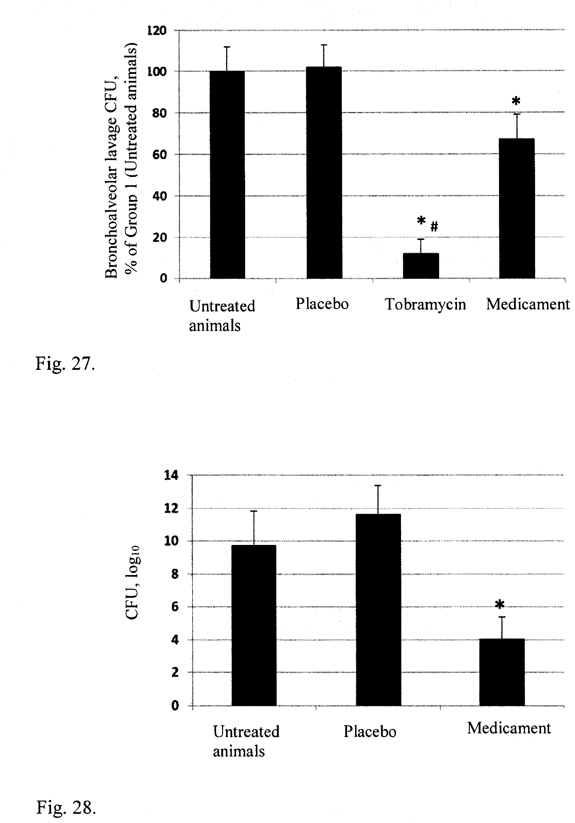

Fig. 27. Bacterial load in the bronchoalveolar lavage of rats on day 5 after intratracheal infection with Pseudomonas aeruginosa (RP73) at a dose of $6.5*10^7$ CFU/ml and administration of placebo, the claimed medicament or tobramycin as therapeutic and preventive therapy. The data are presented as % of the value obtained in Group 1 (Untreated animals), M±SE. * p < 0.05 vs the values obtained in group 1 (Untreated animals) and Group 2 (Placebo); # p < 0.05 vs the values obtained in group 4 (Medicament).

Fig. 28. Bacterial load in the subcutaneously implanted chamber fluids in mice on day 4 after in vivo infection with Porphyromonas gingivalis (ATCC 33277) at a dose of $10^8$ CFU/animal. The data are presented as M±SE. * p < 0.05 vs the values obtained in Group 1 (Untreated animals) and Group 2 (Placebo).

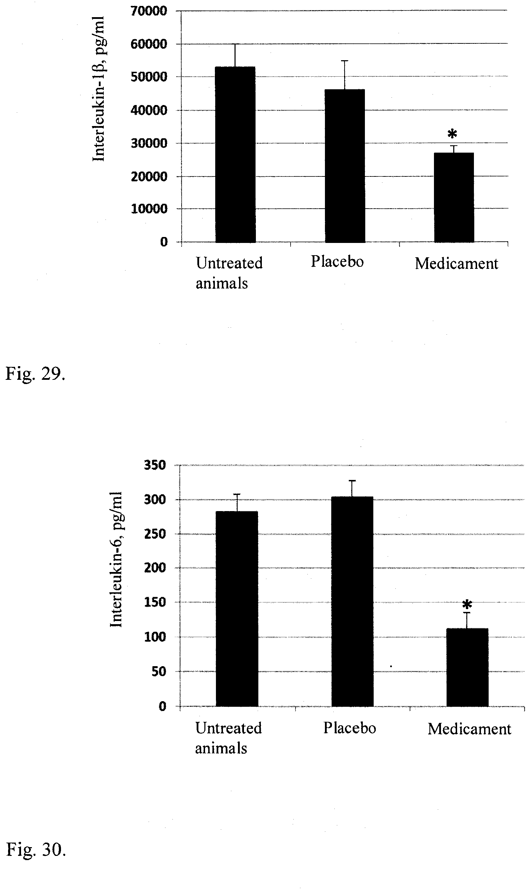

Fig. 29. The interleukin-1β concentration in the subcutaneously implanted chambers in mice on day 4 after in vivo infection with Porphyromonas gingivalis (ATCC 33277) at a dose of $10^8$ CFU/animal. The data are presented as M±SE*. p < 0.05 vs the values obtained in Group 1 (Untreated animals) and Group 2 (Placebo).

Fig. 30. The interleukin-6 concentration in the subcutaneously implanted chambers in mice day 4 after in vivo infection with Porphyromonas gingivalis (ATCC 33277) at a dose of $10^8$ CFU/animal. The data are presented as M±SE*. p < 0,05 vs the values obtained in Group 1 (Untreated animals) and Group 2 (Placebo).

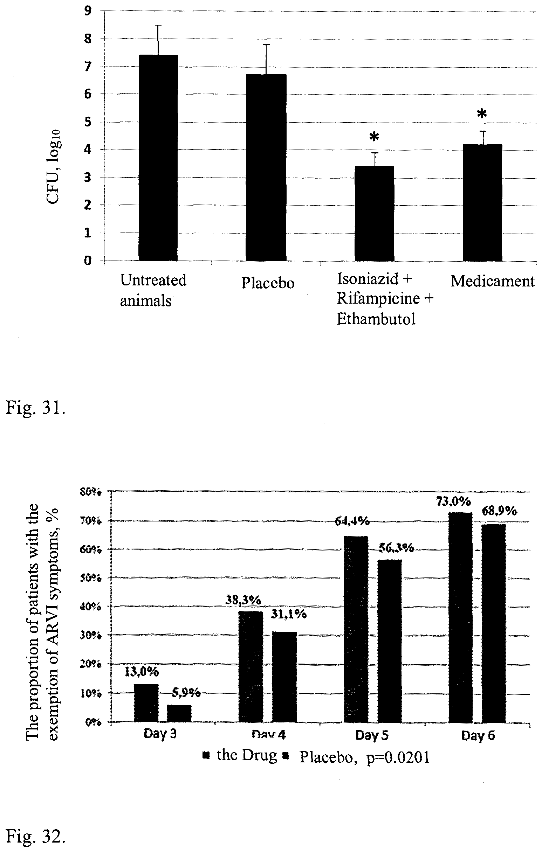

Fig. 31. Bacterial load in mice lungs 4 weeks after infection with Mycobacterium tuberculosis (H37Rv) at a dose of $5*10^6$ CFU/animal and treatment with placebo, the claimed medicament or isoniazid + rifampicine + ethambutol azithromycin combination. The data are presented as M±SE. * p < 0.05 vs the values obtained in Group 1 (Untreated animals) and Group 2 (Placebo).

Fig.32. The proportion of patients with the exemption of ARVI symptoms (clinically diagnosed, including PCR confirmed). PP analysis data.

Example 1.

Antituberculosis activity of the claimed medicament against Mycobacterium tuberculosis induced in guinea pigs.

The aim of the study is to investigate the antituberculotic activity of the medicament against Mycobacterium tuberculosis in a guinea pig model of experimental tuberculosis as compared to the anti-tuberculosis agent Isoniazid. The medicament of this invention contains a technology-processed product resulting from serial dilutions of the stock substance of antibodies to HLA-DRB1 (C12C150 aqueous-alcoholic dilution of monoclonal antibodies to HLA-DRB1 at a ratio of 2:3) and a technology-processed product resulting from serial dilutions of the stock substance of antibodies to β2-MG (C12C150 aqueous dilution of monoclonal antibodies to β2-MG at a ratio of 2:3) at a volume ratio of 1:2:

A) a technology-processed product resulting from serial dilutions of the stock substance of monoclonal antibodies to the β1 domain of the major histocompatibility complex class II molecule (HLA-DRB1) that is obtained by combining two different successive dilutions of the stock (matrix) solution of monoclonal antibodies to HLA-DRB1 in an aqueous-alcoholic solvent (2.5 mg/ml). The following dilutions were subject to mixing at a volume ratio of 2:3:

1) a stock (matrix) solution diluted $100^{12}$ times which is equivalent to a C12 dilution, with shaking of each dilution.
2) a stock (matrix) solution diluted $100^{150}$ times which is equivalent to a C150 dilution, with shaking of each dilution.

B) a technology-processed product resulting from serial dilutions of the stock substance of monoclonal antibodies to β2-microglobulin (β2-MG) that is obtained by combining two different successive dilutions of the stock (matrix) solution of monoclonal antibodies to β2-MG in an aqueous solvent (1.0 mg/ml). The following dilutions were subject to mixing at a volume ratio of 2:3:

1) a stock (matrix) solution diluted $100^{12}$ times which is equivalent to a C12 dilution, with shaking of each dilution.
2) a stock (matrix) solution diluted $100^{150}$ times which is equivalent to a C150 dilution, with shaking of each dilution.

Experimental groups:

Group 1 - intact control, non-infected animals (n=7);
Group 2 - negative control (infected untreated animals) (n=7);
Group 3 - animals receiving placebo (purified water) orally twice daily for 5 days before infection and 6 days a week for two months after 2 weeks of infection in the volume of 4 ml/kg (n=14);
Group 4 - animals receiving placebo (purified water) orally twice daily for 5 days before infection and 6 days a week for two months after 2 weeks of infection in the volume of 4 ml/kg as well as Isoniazid at a dose of 10 mg/kg in the volume 0.4 ml 6 days a week for two months after 2 weeks of infection (n=14);
Group 5 - animals receiving medicament orally twice daily for 5 days before infection and 6 days a week for two months after 2 weeks of infection in the volume of 4 ml/kg (n=14);

Study design:

The duration of this study was 196 days (6.5 months): September-December - treatment and follow up of the experimental animals; January-March - analysis of necropsy specimens by microbiological and histological techniques. Physical and visual examination, health assessment and mortality monitoring were carried out on a daily basis before feeding.

The test items were administered orally to guinea pigs via a syringe without a needle in the total volume of 8 ml/kg twice daily for 5 days before infection and 6 days a week (every day except Sunday) for 2 months after infection. The dose of the test items was adjusted once a week based on the animal body weights. Isoniazid suspended in water and starch was administered to animals at the same time intervals following the same regimen as the test items. The isoniazid dose was 10 mg/kg of body weight in 0.4 ml of a water-starch slurry.

Mortality, body weight, M.tuberculosis growth in media and histological changes in the organs of animals were evaluated in all groups of guinea pigs throughout the study.

Study results:

Table 1. Evaluation of organ involvement in guinea pigs.

| No. | Evaluation criteria | Group, M±m* | | | | |
|---|---|---|---|---|---|---|
| | | 1 intact control | 2 negative control | 3 placebo | 4 placebo +isoniazid | 5 medicament |
| 1 | Life expectancy, units | 3.0±0 | 2.3±0.3 | 1.9±0.2 | 2.7±0.09 | 3±0 |
| 2 | Difference between initial and final body weight of animals, g. | 153.7±16.7 | 13.1±2.7 | 38.0±23.3 | 180.6±28.4 | 200.0±11.6 |
| 3 | Macroscopic lesions in lungs, units. | 0±0 | 3.5±0.4 | 3.6±0.1 | 0.2±0.1 | 0.1±0.1 |
| 4 | MTB number in lung tissue smears stained by the Ziehl-Neelson staining method, units. | 0±0 | 3.6±0.4 | 2.8±0.3 | 0±0 | 0±0 |
| 5 | Isolation of MTB when lung tissue homogenates are inoculated on solid culture media, units. | 0±0 | 2.4±0.3 | 2.3±0.2 | 0±0 | 0±0 |
| 6 | Macroscopic changes in lymph nodes at the site of administration, units. | 0±0 | 1.8±0.4 | 1.4±0.2 | 2.07±0.3 | 1.9±0.07 |
| 7 | Macroscopic changes in mediastinal lymph nodes, units. | 0±0 | 0.5±0.1 | 0.6±0 | 0.85±0.09 | 0.96±0.03 |
| 8 | Macroscopic changes in the liver, units. | 0±0 | 3.3±0.1 | 2.8±0.4 | 0.03±0.03 | 0.04±0.02 |
| 9 | Macroscopic changes in the spleen, units. | 0±0 | 3.1±0.5 | 2.3±0.4 | 0.12±0.07 | 0.09±0.06 |
| Note: M±m* - the mean value of the criteria with the standard error of the mean per group | | | | | | |

Conclusion:

Based on the study results obtained, it can be concluded that the Isoniazid group and the group of the claimed Medicament demonstrated equal positive therapeutic effects. Histological examination of animal organs in the two groups revealed the signs of therapeutic pathomorphism. The claimed medicament also demonstrated efficacy against lung infections.

Example 2.

Antibacterial activity of the claimed medicament against Streptococcus pyogenes in mice with and without antibiotic.

This is a blind placebo-controlled study. The aim of the study is to assess the efficacy of the claimed medicament with or without linezolid (synthetic antibiotic) or telithromycin (ketolide antibiotic) against Streptococcus pyogenes in a model of soft tissue infection in neutropenic mice. The medicament of this invention contains a technology-processed product resulting from serial dilutions of the stock substance of antibodies to HLA-DRB1 (C4D20 aqueous-alcoholic dilution of monoclonal antibodies at a ratio of 3:2, (2.5 mg/ml)) and a technology-processed product resulting from serial dilutions of the stock substance of antibodies to β2-MG (C10C150 aqueous-alcoholic dilution of natural antibodies at a ratio of 1:2, (2.5 mg/ml)) at a volume ratio of 2:3.

Experimental groups:

Group 1 - negative control female mice were infected with S. pyogenes and did not receive the medicament or antibiotic during the study period; n = 3;

Group 2 - female mice received placebo (glycine buffer) orally every day in the volume of 20 ml/kg for 5 days before infection and on the day of infection with S. pyogenes; n = 6;

Group 3 - female mice received placebo (glycine buffer) orally every day in the volume of 20 ml/kg for 5 days before infection and on the day of infection with S. pyogenes; in combination with linezolid (50 mg/kg) at 2 and 14 hours after infection; n = 6;

Group 4 - female mice received placebo (glycine buffer) orally every day in the volume of 20 ml/kg for 5 days before infection and on the day of infection with S. pyogenes; in combination with telithromycin (10 mg/kg) at 2 and 14 hours after infection; n = 6.

Group 5 - female mice received the medicament orally every day in the volume of 20 ml/kg for 5 days before infection and on the day of infection with S. pyogenes; n = 6;

Group 6 - female mice received the medicament orally every day in the volume of 20 ml/kg for 5 days before infection and on the day of infection with S. pyogenes; in combination with linezolid (50 mg/kg) at 2 and 14 hours after infection; n = 6;

Group 7 - female mice received the medicament orally every day in the volume of 20 ml/kg for 5 days before infection and on the day of infection with S. pyogenes; in combination with telithromycin (10 mg/kg) at 2 and 14 hours after infection; n = 6;

Study design:

The duration of the study was 7 days. Neutropenia was induced in mice with two intraperitoneal injections of cyclophosphamide (150 mg/kg 4 days before infection and 100 mg/kg 1 day before infection). The immunocompromised mice did not receive any further immune suppression during the study.

The test samples were administered orally at a dose of 20 ml/kg for 5 days before infection and at 2 and 14 hours after infection (day of infection). On the day of infection, mice were infected with S. pyogenes (ATCC BAA-2469) at a dose of 5.4 lg CFU by intramuscular injection into the thigh. Besides, at 2 and 14 hours after infection, animals received antibiotics - linezolid at 50 mg/kg and telithromycin at 10 mg/kg, to which S. pyogenes BAA-2469 is susceptible and resistant, respectively.

Animal survival, clinical signs of infection and body weight were evaluated in all groups of mice throughout the study.

Study results:

Two hours after infection, femoral muscles were collected from untreated mice (Group 1) for bacterial burden analysis (CFU/g, $\log_{10}$). In other groups, femoral muscles were collected 24 hours after infection to evaluate bacterial burden. The data obtained are given in Table 2.

Table 2. Mean bacterial burden in the femoral tissue (CFU/g, log10)

| Group | 2 hours post infection | 24 hours post infection |
|---|---|---|
| 1 (control) | 5,2 | - |
| 2 (placebo) | - | 9.1 |
| 3 (placebo +L) | - | 5.5 |
| 4 (placebo +T) | - | 8.4 |
| 5 (medicament) | - | 6.5 |
| 6 (medicament + L) | - | 3.7 |
| 7 (medicament + T) | - | 7.1 |

Conclusion:

A statistically significant decrease in the mean bacterial burden was recorded for a combination with linezolid in the test groups (Groups 3 and 6) 24 hours after infection. Thus, the claimed medicament shows efficacy against musculoskeletal infections, and, when co-administered with antibiotics, it was found to enhance their efficacy.

Example 3.

The efficacy of the claimed medicament alone and in combination with antibiotic against E.coli in a murine model of urinary tract infection.

The aim of the study is to evaluate the effects of the test medicament with and without antibiotic Norfloxacin in an experimental model of E.coli infection in mice. The medicament of this invention contains technology-processed products resulting from serial dilutions of the stock substances of antibodies to HLA-DRB1 (C12C30C50 aqueous dilution of polyclonal antibodies at a ratio of 1:1:1, (2.5 mg/ml)) and antibodies to $\beta$2-MG (C12C30C50 aqueous dilution of monoclonal antibodies at a ratio of 1:1:1, (2.5 mg/ml)) at a volume ratio of 1:1.

Experimental groups:

Group 1 - male and female mice (n=10) received the claimed medicament at 20 ml/kg every day via oral gavage for 5 days before infection and for 7 days after infection as well as at 1 hour after infection by intraperitoneal route (volume 10 ml / kg).

Group 2 - male and female mice (n=10) received the claimed medicament at 20 ml/kg every day via oral gavage for 5 days before infection and for 7 days after infection as well as Norfloxacin at 5 mg/kg (volume 10 ml / kg) by intraperitoneal route 1 hour after infection.

Group 3 - control mice of both sexes (n=10) received glycine buffer at 20 ml/kg every day via oral gavage for 5 days before infection and for 7 days after infection as well as at 1 hour after infection by intraperitoneal route (volume 10 ml/kg).

Group 4 - male and female mice (n=10) received glycine buffer at 20 ml/kg every day via oral gavage for 5 days before infection and for 7 days after infection as well as Norfloxacin at 5 mg/kg (volume 10 ml / kg) by intraperitoneal route 1 hour after infection.

Study design:

Mice were infected by intraurethral inoculation of E.coli UTI89 (2.107 CFU/mouse) into the bladder (Day 0).

One, three and six days after infection, urine was collected to quantify CFU. Seven days after infection, 10 animals from each group were sacrificed and kidneys and bladders were collected for bacterial burden analysis. The body weight was recorded every 2 days during the experiment.

Study results:

The body weight demonstrated positive changes in all groups, indicating that the test samples were well tolerated by animals.

The bladder weight was significantly lower in Group 2 (medicament +norfloxacin) compared to Group 4 (glycine buffer + norfloxacin). This may suggest that the claimed medicament combined with antibiotics can reduce swelling of the bladder.

The claimed medicament in combination with norfloxacin (Group 2) significantly decreased the bladder bacterial load. Thus, this combination may have potential as a therapeutic regimen (Fig. 1, 2).

No statistically significant differences in the kidney weights were observed between the groups.

The claimed medicament combined with norfloxacin (Group 2) significantly decreased the kidney bacterial load as compared to glycine buffer (Group 3). Thus, this combination may have potential as a therapeutic regimen (Fig. 3,4).

From Day 3, a urine specimen from Group 2 (medicament+norfloxacin) showed reduced bacterial burden compared to Group 4 (glycine buffer + norfloxacin). A urine specimen from Group 3 (glycine buffer) had a higher number of CFU at all time points as compared to Group 1 (medicament) (Fig. 5).

Conclusion:

Thus, the claimed medicament combined with antibiotic Norfloxacin exerts antibacterial effects against E.coli in a murine model of urinary tract infection.

Example 4.

Antibacterial activity of the claimed medicament with and without antibiotic against Salmonella enteritidis rif 92 in chickens.

This is a blind placebo-controlled study. The aim of the study was to investigate the effects of the claimed medicament against Salmonella enteritidis rif92 in chickens. The medicament of this invention contains equal amounts of two technology-processed products resulting from serial dilutions (C12C30C50 dilutions) of the stock substances of affinity purified polyclonal antibodies, i.e. 1) antibodies to the $\beta$1 domain of the major histocompatibility complex class II molecule and 2) antibodies to $\beta$2-microglobulin of the major histocompatibility complex class I molecule:

A) a technology-processed product resulting from serial dilutions of the stock substance of polyclonal antibodies to the $\beta$1 domain of the major histocompatibility complex class II molecule (HLA-DRB1) that is obtained by combining three different successive dilutions of the stock (matrix) solution of polyclonal antibodies to HLA-DRB1 in an aqueous solvent (2.5 mg/ml). The following dilutions were subject to mixing at a ratio of 1:1:1:

1) a stock (matrix) solution diluted $100^{12}$ times which is equivalent to a C12 dilution, with shaking of each dilution.
2) a stock (matrix) solution diluted $100^{30}$ times which is equivalent to a C30 dilution, with shaking of each dilution.
3) a stock (matrix) solution diluted $100^{50}$ which is equivalent to a C50 dilution, with shaking of each dilution.

B) a technology-processed product resulting from serial dilutions of the stock substance of polyclonal antibodies to $\beta$2-microglobulin ($\beta$2-MG) that is obtained by combining three different successive dilutions of the stock (matrix) solution of polyclonal antibodies to $\beta$2-MG in an aqueous solvent (2.5 mg/ml). The following dilutions were subject to mixing at a volume ratio of 1:1:1:

1) a stock (matrix) solution diluted $100^{12}$ times which is equivalent to a C12 dilution, with shaking of each dilution.
2) a stock (matrix) solution diluted $100^{30}$ times which is equivalent to a C30 dilution, with shaking of each dilution.
3) a stock (matrix) solution diluted $100^{50}$ which is equivalent to a C50 dilution, with shaking of each dilution.

Experimental groups:

Group 1 - male and female chickens (n=15) infected with Salmonella enteritidis received the medicament of this invention intragastrically between day 4 and day 9 of age at a dose of 0.2 ml/animal/day.

Group 2 - male and female chickens (n=15) infected with Salmonella enteritidis received the medicament of this invention intragastrically between day 4 and day 9 of age at a dose of 0.2 ml/animal/day and ciprofloxacin (Ciprovet, OOO Research Innovation Center "Agrovetzashchita", antibiotic) intragastrically between day 5 and day 9 of age at a dose of 0.5 mg/kg body weight (ED50). The pharmaceutical composition of this invention and the antibiotic were administered at one hour interval. As the body weight of chickens increased every day during the study, the volume of the antibiotic was calculated on a daily basis before administration.

Group 3 - male and female chickens (n=15) infected with Salmonella enteritidis received placebo (purified water) intragastrically between day 4 and day 9 of age at a dose of 0.2 ml/animal/day.

Group 4 - male and female chickens (n=15) infected with Salmonella enteritidis received placebo (purified water) intragastrically between day 4 and day 9 of age at a dose of 0.2 ml/animal/day and ciprofloxacin intragastrically between day 5 and day 9 of age at a dose of 0.5 mg/kg of body weight (ED50). The test sample and the antibiotic were administered at an interval of one hour.

Group 5 - intact control animals - non-infected and untreated chickens of both sexes (n=15).

Study design:

The duration of the study was 12 days (day 1 to day 12 of age). Chickens were quarantined for the first two days. On Day 3, all birds, with the exception of chickens from Group 5, were infected with a doubled lethal dose of Salmonella enteritidis (serovar rif92, $2.5 \times 10^9$ CFU/g in the volume of 0.5 ml/animal) by intraperitoneal route. Between day 4 and day 9 of age, chickens were given either the claimed medicament or purified water. Between day 5 and day 9, chickens received Ciprovet (Groups 2 and 4).

The following parameters were examined throughout the study: survival rate; body weight; feed conversion efficiency (the weight of feed intake by the end of the experiment normalized to weight gained by the animal); and the presence and concentration of S. enteritidis rif92 in the gastrointestinal tract and liver of chickens.

Statistical analysis:

Statistical analysis of the data obtained was carried out using two-way generalized linear models (gamma distribution with data normalized by 100 was used for percentage values and log transformation for the other parameters) in combination with the post-hoc Tukey test and the Kruskal-Wallis test in combination with the post-hoc Wilcoxon test. The differences were considered statistically significant if $p < 0.05$.

Study results:

According to the results obtained, the survival rate in all experimental groups was 100%. The maximum live weight at the end of the study was recorded in broilers receiving the claimed medicament (Group 1), exceeding that in the corresponding placebo group (Group 3) and intact group (Group 5) by 33.9 g and 38.7 g, respectively. Feed conversion efficiency was similar between the groups. The basic parameters of hematological examination did not show any difference between the groups.

Summary data on Salmonella counts in the liver and intestinal contents per group at the end of the experiments are given in Table 3.

Table 3. Presence of Salmonella following slaughter and antibacterial activity index (AAI)

| Group number | Presence of Salmonella cells in the liver | | Presence of Salmonella cells in the intestinal contents | | AAI |
| --- | --- | --- | --- | --- | --- |
| | % non-recovered chickens of the total number of chickens in a group | CFU/g in infected animals (median) | % non-recovered chickens of the total number of chickens in a group | CFU/g in infected animals (median) | |
| 1 (Medicament) | 13.3 | 18.5 | 13.3 | $5.5 \times 10^3$ | 10.6 |
| 2 (Medicament +CF) | 6.7 | 56 | 26.7 | $5 \times 10^2$ | 3.5 |
| 3 (Placebo) | 20 | 195.7 | 46.7 | $2.3 \times 10^3$ | - |
| 4 (Placebo+CF) | 6.7 | 200 | 33.3 | $3.8 \times 10^3$ | - |
| 5 (Intact control) | - | - | - | - | - |

The antibacterial activity index (AAI) was calculated using the formula: $$\text{AAI} = \frac{Bc}{Bt}$$, where Bc - the number of bacterial cells in the organ homogenates in the control group by the end of the follow-up period; Bt - the number of bacterial cells in the organ homogenates in the test group by the end of the follow-up period [Okhapkina V.Y., Pyatkova N.V., Fedotov A.K. Express efficacy assessment method for antibacterial agents in experimental brucellosis // Challenges of Highly Infectious Diseases, 2016; 2:79-82.].

According to the data from Table 1, the lowest Salmonella count in the liver was recorded in Group 2 and Group 4, while the highest Salmonella count was observed in Group 3. Meanwhile, the AAI for the medicament of this invention (Group 1) (10.6) significantly exceeded that for its combination with the antibiotic (3.6). In Groups 1 and 2, the bacterial load in the liver was 10.6 and 3.5 times lower than that in Group 3 and Group 4, respectively, suggesting the presence of protective effects of the medicament on the liver in terms of Salmonella cell count.

Conclusion:

1. The claimed medicament demonstrated efficacy (antibacterial action) which was characterized by a decrease in the number of sick chickens by 33.4 % compared to the placebo group and a 10.6-fold reduction in the liver bacterial burden in the medicament group compared to the placebo group. This suggests the presence of protective effects of the medicament on the liver in terms of Salmonella cell count. The claimed medicament is also effective against intestinal infections.

2. The medicament co-administered with the antibiotic at ED50 to chickens enhanced the efficacy of the latter, as a noticeable reduction in the bacterial burden in the gastrointestinal tract was observed at slaughter as compared to the medicament group and placebo group. Thus, it was confirmed that an effective dose of the antibiotic could be reduced, when co-administered with the claimed medicament.

Example 5.

Antibacterial activity of the claimed medicament with and without antibiotic against Escherichia coli CN 160 in mice.

This is a blind placebo-controlled study. The aim of the study is to evaluate pharmacological effects of the test medicament in combination with enrofloxacin therapy (Baytril, Bayer) in experimental infection caused by E.coli CN 160 in mice. The medicament of this invention contains equal amounts of two technology-processed products resulting from serial dilutions (C12C30C50 dilutions) of the stock substances of affinity purified polyclonal antibodies, i.e. 1) antibodies to the β1 domain of the major histocompatibility complex class II molecule (2.5 mg/ml) and 2) antibodies to β2-microglobulin of the major histocompatibility complex class I molecule (2.5 mg/ml).

Experimental groups:

Group 1 - male and female mice (n=10) received the claimed medicament orally once a day for 5 days before infection, 1 hour after infection and for the following 14 days at a dose of 10 ml/kg/day.

Group 2 - control mice of both sexes (n=10) received placebo (purified water) orally once a day for 5 days before infection, 1 hour after infection and for the following 14 days at a dose of 10 ml/kg/day.

Group 3 - male and female mice (n=10) received Baytril (antibacterial agent) by intramuscular injection at a dose of 1.38 mg/kg for 3 days before infection and for 3 days after infection.

Group 4 - male and female mice (n=10) received the claimed medicament orally once a day for 5 days before infection, 1 hour after infection and for the following 14 days at a dose of 10 ml/kg/day as well as Baytril (antibacterial agent) co-administered by intramuscular injection at a dose of 1.38 mg/kg for 3 days before infection and 3 days after infection.

Study design:

The test items were administered orally once a day for 5 days before infection with E.coli, 1 hour after infection (Day 6) and the following 14 days during the follow-up period (for a total of 20 days). On Day 6, mice were infected by a single intraperitoneal injection at a dose of 2 LD100 that was pre-determined in a preliminary study (LD100=$7.3 \times 10^8$ CFU/mouse). The antibacterial agent Baytril was administered by intramuscular injection for 3 days before infection and 3 days after infection (for a total of 6 days) at the pre-determined EF50, i.e. 1.38 mg/kg.

Mortality rates were assessed in all groups of mice throughout the study.

Statistical analysis:

Statistical analysis of the findings was carried out using the Kaplan Meier curves followed by pairwise comparisons of the log-rank tests. The differences were considered statistically significant if $p<0.05$.

Study results:

All animals in each group, with the exception of mice receiving a combination of the claimed medicament and Baytril (Group 4), died within four days after infection with a lethal dose of Escherichia coli (Fig.6). The highest mortality rates (70-80 %) were observed in the first two days after infection.

Conclusion:

The use of the claimed medicament in combination with Baytril at ED50 in mice infected with 2LD100 of E.coli CN 160 prevented death of 50% of animals, indicating a significant antibacterial action of this combination against a doubled lethal dose of E.coli CN-160. Thus, in this study, the co-administration of the claimed medicament of this invention and antibiotic was found to enhance the pharmacological action of the antibiotic. Besides, this combination was also shown to reduce an effective dose of the antibiotic.

The claimed medicament was also found to be effective against systemic infections, including sepsis.

Example 6.

Efficacy of the medicament against infection of mice with Klebsiella pneumoniae BAA-1705 strain resistant to amoxicillin/clavulanic acid.

The aim of the study is to investigate the antibacterial action of the claimed medicament combined with amoxicillin+clavulanic acid (AMC) against an AMC-resistant strain of Klebsiella pneumoniae. The medicament of this invention contains equal amounts of two technology-processed products resulting from serial dilutions (C12C30C50 dilutions) of the stock substances of affinity purified polyclonal antibodies, i.e. 1) antibodies to the $\beta1$ domain of the major histocompatibility complex class II molecule (2.5 mg/ml) and 2) antibodies to $\beta2$-microglobulin of the major histocompatibility complex class I molecule (2.5 mg/ml).

Study No.1.

Experimental groups:

Group 1 - male mice received the claimed medicament + AMC combination intragastrically at a dose of 25 mg/kg in 10 ml/kg 0.9% NaCl for 5 days before and 1 hour after infection at a dose of 10 ml/kg/day (n=15);
Group 2 - control male mice received purified water intragastrically for 5 days before and 1 hour after infection at a dose of 10 ml/kg/day (n=15);
Group 3 - male mice received the purified water + AMC combination intragastrically at a dose of 25 mg/kg in 10 ml/kg 0.9% NaCl for 5 days before and 1 hour after infection at a dose of 10 ml/kg/day (n=15);
Group 4 - male mice received AMC by intraperitoneal route 1 hour after infection at a dose of 25 mg/kg in 10 ml/kg 0.9% NaCl (n=15);
Group 5 - positive control male mice received Gentamicin (Sigma) by intraperitoneal route at a dose of 15 mg/kg in 10 ml/kg 0.9% NaCl (n=15).

Study design:

A model of bacterial pneumonia in neutropenic mice was set up. Pneumonia was induced by intranasal administration of Klebsiella pneumoniae (BAA-1705 strain with AMC resistance, $10^6$ CFU/mouse). To induce neutropenia, mice received cyclophosphamide four days (Day -4) and one day (Day -1) before infection by intraperitoneal route at 150 mg/kg and 100 mg/kg, respectively.
Bacterial burden in the lungs and body weight as a parameter of general health were assessed. Survival was recorded twice a day.
Statistical analysis of the data was carried out using the Kruskal-Wallis test in combination with the post-hoc Tukey test and Kaplan Meier curves followed by pairwise comparisons of the log-rank tests. The differences were considered statistically significant if $p<0.05$.

Study results:

None of the test items produced effects on the lung weights of infected animals and CFU number. Body weight loss triggered by infection was observed in all test groups (Fig.7). In Group 5 (gentamicin) and Group 1 (AMC+ medicament), body weight evolution was observed from 54 h of the follow-up period.

Mortality rates in all groups were recorded at 48h-54h of the follow-up period (Fig.8). All animals in the control group and AMC+Control group died, which confirmed the resistance of Klebsiella pneumoniae (BAA-1705) to the antibiotic. Gentamicin and AMC+ medicament treatment improved mice survival, 70% and 50% of animals survived, accordingly.

Conclusion:

Thus, the study demonstrated significant antibacterial effects of the claimed medicament in combination with AMC against the Klebsiella pneumoniae strain with AMC resistance. This effect was comparable to that of the comparator drug gentamicin, to which the strain is resistant.

Study No.2.

Experimental groups:

Group 1 - male mice received the claimed medicament intragastrically at a dose of 20 ml/kg/day for 5 days before and 2 days after infection (n=5) as well as AMC at a dose of 25 mg/kg in 10 ml/kg 0.9% NaCl by intraperitoneal route 1 hour after infection;
Group 2 - control male mice received purified water intragastrically at a dose of 20 ml/kg/day for 5 days before and 2 days after infection (n=5) as well as AMC at a dose of 25 mg/kg in 10 ml/kg 0.9% NaCl by intraperitoneal route 1 hour after infection.

Study design:

A model of bacterial pneumonia was set up in animals by administering Klebsiella pneumoniae (BAA-1705 strain with AMC resistance, $10^7$ CFU/mouse). Forty-eight hours after infection lung tissues from mice of both groups were homogenized and used to prepare $1 \times 10^4$ CFU/ml Klebsiella pneumoniae. The bacteria ($5 \times 10^4$ CFU/well) were then incubated with 2.5-1080 $\mu$g/ml AMC for 24 hours at 37°C. At the end of the experiment, the 50% inhibitory concentration of AMC (IC50) was estimated by spectrophotometry for each group as well as values for 100% bacteria growth inhibition, when exposed to AMC at 1080 $\mu$g/ml. e experiment was performed in duplicate. An additional experiment was carried out to assess the effects of the AMC IC50 on intact bacteria (refer to Fig.9).
Statistical analysis of the data obtained was carried out using the one-way and two-way ANOVA followed by pairwise comparisons of the log-rank tests. The differences were considered statistically significant if p<0.05.

Study results:

AMC displayed a 100% inhibitory concentration at 1080 $\mu$g/ml confirming the resistance of Klebsiella pneumoniae (BAA-1705) to AMC. In the in vitro study, the co-administration of the claimed medicament and AMC reduced the AMC IC50 by 27%, i.e. the therapy applied decreased the AMC concentration that induced 50% bacterial growth inhibition. The analysis of the effects of the test combination on the fixed AMC concentration of 1080 $\mu$g/ml (100% growth inhibition concentration) demonstrated similar results, i.e. the maximum inhibitory effect of AMC increased by 26% (Fig.9).

Conclusion:

Thus, the study demonstrated a significant antibacterial action of the claimed medicament and AMC combination against AMC-resistant Klebsiella pneumoniae strain, which resulted in reduced bacterial resistance to AMC. The claimed medicament was shown to be effective against lung infection.

Example 7.

Anti-inflammatory activity of the claimed medicament in a model of carrageenan induced inflammation in rats.
The aim of the study is to assess the anti-inflammatory activity of the claimed medicament in a model of carrageenan induced inflammation in rats as compared to the anti-inflammatory drug Indomethacin (Sigma). The medicament of this invention contains equal amounts of two technology-processed products resulting from serial dilutions (C12C30C50 dilutions) of the stock substances of affinity purified polyclonal antibodies, i.e. 1) antibodies to the $\beta$1 domain of the major histocompatibility complex class II molecule (2.5 mg/ml) and 2) antibodies to $\beta$2-microglobulin of the major histocompatibility complex class I molecule (2.5 mg/ml).

Experimental groups:

Group 1 - male rats received the claimed medicament intragastrically at a dose of 7.5 ml/kg/day for five days before induction of inflammation (the last administration -1 h prior to induction) (n=10);
Group 2 - control male rats received purified water intragastrically at a dose of 7.5 ml/kg/day for five days before induction of inflammation (the last administration -1 h prior to induction) (n=10);
Group 3 - positive control male rats received indomethacin intragastrically at a dose of 10 mg/kg (in 7.5 ml/kg/day purified water.

Study design:

Inflammatory response was induced in animals by subplantar injection (into the plantar aponeurosis of the left hind paw) of 0.1 ml 1 % carrageenan solution.
The severity of inflammatory response was assessed by estimating oedema weight gain in mg calculated as a difference between the paw weights with and without oedema.
Statistical analysis of the findings was carried out using the Kruskal-Wallis test in combination with the post-hoc Tukey test. The differences were considered statistically significant if p<0.05.

Study results:

The claimed medicament decreased oedema weight by 39.1% as compared to the control, while indomethacyn, by 57.9% (Fig. 10).

Conclusion:

Thus, the claimed medicament, which is a composition of a technology-processed product resulting from serial dilutions of the stock substance of antibodies to HLA DRB1 and a technology-processed product resulting from serial dilutions of the stock substance of antibodies to β2-MG, exhibited pronounced anti-inflammatory effects in a model of carrageenan-induced inflammation in rats by reducing paw oedema weight.

Example 8.

Antibacterial activity of the claimed medicament against streptomycin- or nalidixic acid-resistant strains of Salmonella enterica (JB271 and JB285, respectively).
This is a blind placebo-controlled study. The aim of the study is to assess the effects of the claimed medicament on the antibiotic sensitivity-resistance index in experimental peritonitis (sepsis) in mice caused by Salmonella enterica serovar Typhimurium LT-2. The medicament of this invention contains technology-processed products resulting from serial dilutions of the stock substances of antibodies to HLA-DRB1 (C12C30C50 dilution of polyclonal antibodies (2 mg/ml) at a ratio of 2:1:3) and antibodies to β2-MG (C12C30C50 dilution of polyclonal antibodies (2 mg/ml) at a ratio of 2:1:3) at a volume ratio of 1:1.

Experimental groups:

Group 1 - female BALB/c mice (n=12) received placebo (purified water) orally at 1, 4 and 8 hours after infection with Salmonella enterica (a 1:1 combination of streptomycin-susceptible Salmonella enterica Typhimurium LT-2 (JB270 strain) and streptomycin-resistant Salmonella enterica Typhimurium LT-2 (JB271 strain), by intraperitoneal route at 5.0 log10 CFU/animal). Dose volume - 10 ml/kg.
Group 2 - female BALB/c mice (n=12) received the claimed medicament orally at 1, 4 and 8 hours after infection with Salmonella enterica (a 1:1 combination of streptomycin-susceptible Salmonella enterica Typhimurium LT-2 (JB270 strain) and streptomycin-resistant Salmonella enterica Typhimurium LT-2 (JB271 strain), by intraperitoneal route at 5.0 log10 CFU/animal). Dose volume - 10 ml/kg.
Group 3 - female BALB/c mice (n=12) infected with Salmonella enterica (a 1:1 combination of streptomycin-susceptible Salmonella enterica Typhimurium LT-2 (JB270 strain) and streptomycin-resistant Salmonella enterica Typhimurium LT-2 (JB271 strain), by intraperitoneal route at 5.0 log10 CFU/animal) as mice from Group 1 and Group 2, which did not receive any agents (untreated animals).
Group 4 - female BALB/c mice (n=12) received placebo (purified water) orally at 1, 4 and 8 hours after infection with Salmonella enterica (a 1:1 combination of nalidixic acid-susceptible Salmonella enterica Typhimurium LT-2 (JB270 strain) and nalidixic acid-resistant Salmonella enterica Typhimurium LT-2 (JB285 strain), by intraperitoneal route at 5.0 log10 CFU/animal). Dose volume - 10 ml/kg.
Group 5 - female BALB/c mice (n=12) received the claimed medicament orally at 1, 4 and 8 hours after infection with Salmonella enterica (a 1:1 combination of nalidixic acid-susceptible Salmonella enterica Typhimurium LT-2 (JB270 strain) and nalidixic acid-resistant Salmonella enterica Typhimurium LT-2 (JB285 strain), by intraperitoneal route at 5.0 log10 CFU/animal). Dose volume - 10 ml/kg.
Group 6 - female BALB/c mice /c (n=12) infected with Salmonella enterica (a 1:1 combination of nalidixic acid-susceptible Salmonella enterica Typhimurium LT-2 (JB270 strain) and nalidixic acid-resistant Salmonella enterica Typhimurium LT-2 (JB285 strain), by intraperitoneal route at 5.0 log10 CFU/animal) as mice from Group 4 and Group 5, which did not receive any agents (untreated animals).

Study design:

The duration of the study was 5 days. Peritonitis (sepsis) was induced in mice by intraperitoneal injection of Salmonella enterica either sensitive or resistant to streptomycin and nalidixic acid at a dose of 5.0 log10 CFU/animal at a ratio of 1:1, i.e. JB270 + JB271 [Strep$^R$] at a ratio of 1:1 or JB270 + JB285 [Nal$^R$] at a ratio of 1:1, respectively).

Test samples were administered orally at a dose of 10 ml/kg 1, 4 and 8 hours after infection.

On Days 2, 3 and 4 after infection, 4 mice per group were euthanized by cervical dislocation and their spleens were collected. The organs collected were homogenized and serially diluted. An amount of 20 $\mu$l of the resultant decimal dilutions was then plated in triplicate on LB agar plates with either of the antibiotics (streptomycin at a dose of 50 $\mu$g/ml or nalidixic acid at a dose of 30 $\mu$g/ml).

After 24 hours, the number of colony forming units (CFU) per dilution was counted and the sensitivity-resistance index per spleen tissue gram for JB270 / JB271 [Strep$^R$] or JB270 / JB285 [Nal$^R$] was calculated for each mouse.

Statistical analysis of the findings was carried out using the two-way repeated measures ANOVA in combination with the post-hoc Tukey test. The differences were considered statistically significant if $p < 0.05$.

Study results:

The experiment with streptomycin-resistant Salmonella enterica (JB271 [Strep$^R$]) and streptomycin-sensitive JB270 strain at 1:1 demonstrated that the absence of treatment (Group 3) did not change the CFU counts for each strain throughout the experiment. Administration of placebo (purified water, Group 1) provided similar results. However, in Group 2 (mice receiving the test medicament), the CFU number of the streptomycin-resistant strain was found to decrease day by day during the study. On Day 4 and 5, the JB270 / JB271 [Strep$^R$] ratio exceeded both baseline values in this group (by 66.7% and 63.6%, respectively) and values in the control groups. When compared to Group 1 (placebo), the values were found to increase as follows: 75% and 72.7% on Day 2, 75% and 72.7% on Day 3, 66.7% and 63.6% on Day 4 and 58.3% and 54.5% on Day 5 of the study. When compared to Group 3 (untreated animals), the values were found to increase as follows: 75% and 72.7% on Day 2, 83,3% and 81,8% on Day 3 and 58,3% and 54,5% on Days 4 and 5 of the study (Fig. 11).

The experiment with nalidixic acid-resistant Salmonella enterica (JB285 [Nal$^R$]) and nalidixic acid-sensitive JB270 strain at 1:1 demonstrated that the absence of treatment (Group 6) did not affect significantly the CFU counts for each strain throughout the experiment. Administration of placebo (purified water, Group 4) provided similar results. However, in Group 5 (mice receiving the test medicament), the CFU number of the nalidixic acid-resistant strain showed a progressive decline from day 2 after infection (Day 3 of the study). By the end of the study, the effects observed reached statistical significance: the JB270 / JB285 [Nal$^R$] ratio exceeded the values obtained in this group by 50% on Day 3 of the study. Besides, the result obtained in Group 5 on Day 5 of the study was also significantly different from that in both control groups throughout the study period. When compared to Group 4 (placebo), the values were found to increase to 70-90%, whereas the difference from Group 6 (untreated animals) varied between 30 and 80% (Fig. 12)

Conclusion:

Thus, the study demonstrated pronounced antibacterial effects of the claimed medicament against Salmonella enterica that resulted in significantly reduced resistance of bacteria to the tested antibiotics - streptomycin and nalidixic acid (i.e. a decrease in the CFU count for JB271 [Strep$^R$] and JB285 [Nal$^R$]).

Example 9.

Efficacy of the medicament in mice infected with Clostridium perfringens (ATCC 13124).

This is a blind, placebo-controlled, comparative study. The aim of the study is to assess the antibacterial effects of the claimed medicament in experimental infection of mice with gas-forming bacteria Clostridium perfringens. The medicament of this invention contains technology-processed products resulting from serial dilutions of the stock substances of antibodies to HLA-DRB1 (C12D50D200 dilution of monoclonal antibodies (1.5 mg/ml) at a ratio of 1:2:3) and antibodies to $\beta$2-MG (C10D30D150 dilution of polyclonal antibodies (1.5 mg/ml) at a ratio of 2:1:3) at a volume ratio of 2:1.

Experimental groups:

Group 1 - female outbred Swiss Webster mice (n=20) received placebo (purified water) orally for 5 days before infection and 2 and 8 hours after infection with Clostridium perfringens, ATCC 13124 (by intraperitoneal route, $10^{10}$ CFU/animal). Dose volume - 10 ml/kg.

Group 2 - female outbred Swiss Webster mice (n=20) received the claimed medicament orally for 5 days before infection and 2 and 8 hours after infection with Clostridium perfringens, ATCC 13124 (by intraperitoneal route, $10^{10}$ CFU/animal). Dose volume - 10 ml/kg.

Group 3 - female outbred Swiss Webster mice (n=20) received tetracycline by intraperitoneal route at a dose of 21 mg/kg 30 minutes as well as 4 and 8 hours after infection with Clostridium perfringens, ATCC 13124 (by intraperitoneal route, $10^{10}$ CFU/animal).

Group 4 - female outbred Swiss Webster mice (n=20) received cefoxicin by intraperitoneal route at a dose of 160 mg/kg 30 minutes as well as 4 and 8 hours after infection with Clostridium perfringens, ATCC 13124 (by intraperitoneal route, $10^{10}$ CFU/animal).

Group 5 - female outbred Swiss Webster mice (n=20) received deionized water by intraperitoneal route in the volume of 0.1 ml/kg 30 minutes as well as 4 and 8 hours after infection with Clostridium perfringens, ATCC 13124 (by intraperitoneal route, $10^{10}$ CFU/animal).

Study design:

The duration of the study was 8 days. Experimental infection in mice was induced by intraperitoneal injection of Clostridium perfringens (ATCC 13124) at a dose of $10^{10}$ CFU/animal.

Test samples were administered orally at a dose of 10 ml/kg for 5 days before infection and 2 and 8 hours after infection. The comparator drugs - cefoxitin at 21 mg/kg and tetracycline at 160 mg/kg - and their control (deionized water, 0.1 ml/kg) were injected by intraperitoneal route 30 minutes as well as 4 and 8 hours after infection.

Animal survival was estimated for each group 12, 24, 48 and 72 hours after infection.

Statistical analysis of the findings was carried out using the Kaplan Meier curves followed by pairwise comparisons of the log-rank tests. The differences were considered statistically significant if $p < 0.05$.

Study results:

The highest death rates among infected mice were observed in both control groups: 24 hours after inoculation of bacteria to the animals, only 5 % of mice were still alive in Group 1 (purified water) and 100% of mice died in Group 5 (deionized water). The therapeutic and preventive administration of the claimed medicament (for 5 days before infection and 2 and 8 hours after infection) significantly increased animal survival. In this group, 85% of animals were alive at 12 hours after infection; 55% - after 24 hours, and 40% - after 48 hours and onwards. In this group, 85% of animals were alive at 12 hours after infection; 55% - after 24 hours, and 40% - after 48 hours and onwards. In the groups of the comparator drugs, i.e. tetracycline and cefoxitin, the number of survived animals at 12, 24, 48 and 72 hours after infection was 95%, 85%, 80%, 80% (no deaths at the last measurement observed) and 65%, 55%, 15%, 15% (no deaths at the last measurement observed), respectively. It should be noted that the antibacterial effects of the claimed medicament were significantly more intensive than those obtained for the cefoxitin group. Nevertheless, both of the drugs exerted lower activity compared to tetracycline (Fig. 13).

Conclusion:

Thus, the study demonstrated a significant antibacterial action of the claimed medicament against Clostridium perfringens (ATCC 13124) that resulted in a significant increase in the survival rate as compared to the two control groups (Groups 1 and 5) and the comparator drug cefoxitin.

Example 10.

Antibacterial activity of the claimed medicament against Chlamydophila pneumonia, CM1 strain.

This is a blind, placebo-controlled, comparative study. The aim of the study is to assess immunotropic and antibacterial effects of the claimed medicament in experimental infection of mice with Chlamydophila pneumonia CM1 strain (a model of lung infection). The medicament of this invention contains technology-processed products resulting from serial dilutions of the stock substances of antibodies to HLA-DRB1 (C20C50C200 dilution of polyclonal antibodies (1.5 mg/ml) at a ratio of 2:1:3) and antibodies to β2-MG (C20C50C200 dilution of polyclonal antibodies (3 mg/ml) at a ratio of 2:1:3) at a volume ratio of 1:2.

Experimental groups:

Group 1 - male C57BL/6J mice (n=21) received placebo (purified water) orally for 3 days before infection and 2, 24, 48 and 72 hours after injection of Chlamydophila pneumonia CM1 strain (by intratracheal route, $1.5*10^6$ IFU/animal (IFU - inclusion-forming units)). Dose volume - 10 ml/kg.

Group 2 - male C57BL/6J mice (n=21) received the claimed medicament orally for 3 days before infection and 2, 24, 48 and 72 hours after injection of Chlamydophila pneumonia CM1 strain (by intratracheal route, $1.5*10^6$ IFU/animal). Dose volume - 10 ml/kg.

Group 3 - male C57BL/6J mice (n=21) received azithromycin subcutaneously at a dose of 10 mg/kg 30 minutes and then every 24 hours (Days 2, 3 and 4) after injection of Chlamydophila pneumonia CM1 strain (by intratracheal route, $1.5*10^6$ IFU/animal).

Group 4 - intact male C57BL/6J mice (n=5) [background control for estimation of cytokine levels].

Study design:

The duration of the study was 15 days. A model of lung infectin was induced in mice by intratracheal administration of Chlamydophila pneumonia (CM1) at a dose of $1.5*10^6$ IFU/mouse.

In Groups 1 and 2, the test samples were administered orally at a dose of 10 ml/kg for 3 days before infection and 2, 24, 48 and 72 hours after injection of bacteria. The comparator drug azithromycin (Group 3) was administered subcutaneously at a dose of 10 mg/kg 30 minutes after infection and then every 24 hours (Days 2, 3 and 4).

Animal survival was evaluated on Days 3, 6 and 12 after infection. On the same days, 4 mice from each of three groups, i.e. Groups 1-3, were sacrificed by cervical dislocation and their lungs were collected. The organs collected were homogenized and the resultant homogenate was used to measure the bacterial load of Chlamydophila pneumonia (CM1) and cytokine levels - IL-1$\beta$, IL-6 and TNF-$\alpha$.

Intact animals whose biospecimens (lung tissue homogenate) were used for measurement of cytokine levels to obtain background values were sacrificed on Day 3 after infection of mice from Groups 1-3.

To estimate the bacterial load, 20 $\mu$l samples of the lung homogenate were plated in triplicate on RPMI1640 medium containing 1 mg/ml cycloheximide and HEp2 cells and incubated for 72 hours. The cells were then washed with PBS, blocked with methanol and stained with FITC-conjugated monoclonal antibodies against genus-specific antigen of Chlamidia (Pathfinder Chlamydia Culture Confirmation System; BIO-RAD, Hercules, USA). The preparations were examined using a fluorescence microscope and infected cells were counted.

Cytokine levels were measured using the ELISA test system by Bender MedSystems (USA) according to the manufacturer's instructions.

Statistical analysis of the findings was carried out using the Kruskal-Wallis test in combination with the post-hoc Tukey test and Kaplan Meier curves followed by pairwise comparisons of the log-rank tests. The differences were considered statistically significant if $p < 0.05$.

Study results:

The intact group demonstrated the 100% animal survival rate.

The highest death rate of infected mice was observed in the control group: on Day 3 of the study, 53% of mice in Group 1 (purified water as a placebo) were alive after inoculation of bacteria, on Day 6 and onwards, 24% of mice. The therapeutic and preventive administration of the claimed medicament (Group 2) (for 3 days before infection and 2, 24, 48 and 72 hours after inoculation of bacteria) significantly increased animal survival. On Day 3 after infection, 72% of animals survived in this group, on Day 6 and onwards, 62% of mice. The highest survival rate (81%) was observed in animals receiving the comparator drug azithromycin (Group 3): deaths were first recorded on Day 3 after infection, with the death rate being 19% (Fig. 14).

The bacterial load determined from the number of infected HEp2 cells after exposure to lung tissue homogenate from mice infected with Chlamydophila pneumonia (CM1) demonstrated that the test parameter linearly decreased in response to the claimed medicament therapy as compared to placebo: the number of infected cells in Group 2 (medicament) was 67%, 58% and 21% on Days 3, 6 and 12 after infection, respectively. For the comparator drug Azithromycin (Group 3), a more pronounced decrease in the bacterial load was observed: up to 15%, 17% and 5% on Days 3, 6 and 12 after infection, respectively (Fig. 15).

The study of the immunotropic activity of the claimed medicament showed that both IL-1$\beta$ and IL-6 levels significantly and linearly reduced in response to the therapeutic and preventive course of the medicament (Group 2) as compared to the control. On Days 3, 6 and 12 after infection, the IL-1$\beta$ concentration was 55%, 21% and 20% of the control levels (Group 1, purified water), and the IL6 concentration was 57%, 34% and 27%, accordingly. In Group 3 (comparator drug Azithromycin), the levels of both interleukins demonstrated a more pronounced linear decline, similarly to the bacterial load values (IL-1β: to 17%, 7% and 6% of the control levels on Days 3, 6 and 12 after infection, respectively; IL-6: to 15%, 8% and 5%, respectively) (Fig. 16, Fig. 17).

The TNF-α levels in the lung tissue of mice did not show any changes in all groups, suggesting the absence of changes in the concentration of this cytokine in the induced experimental model of lung infection, and therefore no biological significance was observed (Fig. 18).

Conclusion:

Thus, the study on a model of lung infection caused by Chlamydophila pneumonia, CM1 strain, demonstrated pronounced and concomitant antibacterial and immunotropic effects of the claimed medicament. The activity revealed resulted in a more than 2.5-fold increase in the animal survival rates by the end of the study as well as a 79% decline in the bacterial load and pro-inflammatory cytokine levels (IL-1β by 80% and IL-6 by 73%) in the lungs.

Example 11.

Antibacterial activity of the claimed medicament against Klebsiella pneumoniae, 8637 strain.

This is a blind, placebo-controlled, comparative study. The aim of the study is to assess the antibacterial effects of the claimed medicament in experimental infection of immunocompromised mice with Klebsiella pneumoniae 8637 strain (neutropenic lung infection model). The medicament of this invention contains technology-processed products resulting from serial dilutions of the stock substances of antibodies to HLA-DRB1 (C30C200 dilution of monoclonal antibodies (3 mg/ml) at a ratio of 2:3) and antibodies to β2-MG (C30C200 dilution of polyclonal antibodies (3 mg/ml) at a ratio of 3:1) at a volume ratio of 1:1.

Experimental groups:

Group 1 - female ICR mice (n=20) received placebo (purified water) orally 4, 6, 8, 24 and 48 hours after infection with Klebsiella pneumoniae, 8637 strain (by intranasal route, 2*10⁷ CFU/animal). Dose volume - 10 ml/kg.

Group 2 - female ICR mice (n=20) received the claimed medicament orally 4, 6, 8, 24 and 48 hours after infection with Klebsiella pneumoniae, 8637 strain (by intranasal route, 2*10⁷ CFU/animal). Dose volume - 10 ml/kg.

Group 3 - female ICR mice ICR (n=20) received the comparator drug imipenem by intraperitoneal route at a dose of 2 mg/kg 4, 24 and 48 hours after infection with Klebsiella pneumoniae, 8637 strain (by intranasal route, 2*10⁷ CFU/animal).

Study design:

The duration of the study was 9 days. A model of lung infection was induced in immunocompromised mice by intranasal inoculation of Klebsiella pneumoniae (8637 strain) at a dose of 2*10⁷ CFU/animal. To induce neutropenia (immunosuppression), animals received cyclophosphamide by intraperitoneal route at a dose of 200 mg/kg 4 days before infection (Day -4).

In Groups 1 and 2, the test samples were administered orally at a dose of 10 ml/kg as a therapeutic regimen, i.e. 4, 6, 8, 24 and 48 hours after inoculation of bacteria. The comparator drug imipenem (Group 3) was administered by intraperitoneal route at a dose of 2 mg/kg 4, 24 and 48 hours after infection.

Animal survival was estimated during 5 days after infection with Klebsiella pneumoniae (8637 strain). Upon the end of the experiment, i.e. on Day 5 after infection, 5 mice per group were sacrificed and their blood, lungs, liver, spleen and kidneys were removed to measure the CFU number. The organs isolated were homogenized and serially diluted. 200 μl of the resultant decimal dilutions of all tissue specimens were plated in triplicate on Mueller-Hinton agar and incubated for 24 hours.

Statistical analysis of the findings was carried out using the one-way ANOVA in combination with the post-hoc Tukey test as well as by comparing the survival curves using the log-rank test with the Benjamini-Hochberg correction for multiple testing. The differences were considered statistically significant if p < 0.05.

Study results:

The highest death rate of infected mice was observed in the control group: on Day 1 of the study, 55% of mice in Group 1 (purified water as a placebo) were alive after inoculation of bacteria, on Day 2, Day 3, Day 4 and onwards, 45%, 20% and 15% of mice, respectively. The treatment with the claimed medicament (Group 2) (4, 6, 8, 24 and 48 hours after inoculation of bacteria) significantly increased animal survival. On Day 1 after infection, 85% of animals survived in this group, on Days 2, 3, 4 and onwards, 70%, 60% and 45% of mice, respectively. The highest survival rate was observed in animals receiving the comparator drug imipenem (Group 3): 85% on Day 1 after infection, 70% on Day 2 and 50% on Day 3 and onwards after infection (Fig.19).

The assessment of the bacterial load in the blood, lungs, liver, spleen and kidneys of immunocompromised mice infected with Klebsiella pneumoniae (8637 strain) demonstrated that the number of CFU decreased in all tissues in response to the claimed medicament therapy as compared to the control (placebo): by 68% in the blood, by 56% in the lungs, by 36% in the liver, by 33% in the spleen, and by 44% in the kidneys. For the comparator drug Imipenem (Group 3), similar effects were observed: the bacterial loads in the blood, lungs, liver, spleen and kidneys declined by 67%, 48%, 40%, 45% and 61%, respectively (Fig. 20).

Conclusion:

Thus, the study performed on a model of lung infection in immunocompromised mice that was induced by administration of cyclophosphamide and Klebsiella pneumoniae (8637 strain) demonstrated pronounced antibacterial effects of the claimed medicament. The activity observed resulted in increased survival rates by 30% by the end of the study compared to placebo, with the bacterial load in the blood, lungs, liver, spleen and kidneys being reduced by at least one third.

Example 12.

Antiviral effects of the claimed medicament on a model of lethal influenza infection caused by influenza A/California/07/09(H1N1)pdm09 strain in white mice.

This is a blind placebo-controlled study. The aim of the study is to investigate the antiviral efficacy of the claimed medicament as compared to Tamiflu™ (Oseltamivir) at its effective dose on influenza A/ California/07/09 (H1N1) pdm09 strain in vivo. The medicament of this invention contains technology-processed products resulting from serial dilutions of the stock substances of a) antibodies to HLA-DRB1 (C12C30C50 dilution at a ratio of 1:3:1), b) antibodies to β2-microglobulin (C12C30C50 dilution at a ratio of 1:3:1), c) antibodies to interferon gamma (C12C30C50 dilution at a ratio of 1:3:1) and d) antibodies to CD4 (C12C30C50 dilution at a ratio of 1:3:1) at a volume ratio of 1:1:1:1:

A) a technology-processed product resulting from serial dilutions of the stock substance of polyclonal antibodies to the β1 domain of the major histocompatibility complex class II molecule (HLA-DRB1) that is obtained by combining three different successive dilutions of the stock (matrix) solution of polyclonal antibodies to HLA-DRB1 in an aqueous solvent (2.5 mg/ml). The following dilutions were subject to mixing at a ratio of 1:3:1:

1) a stock (matrix) solution diluted $100^{12}$ times which is equivalent to a C12 dilution, with shaking of each dilution.
2) a stock (matrix) solution diluted $100^{30}$ times which is equivalent to a C30 dilution, with shaking of each dilution.
3) a stock (matrix) solution diluted $100^{50}$ times which is equivalent to a C50 dilution, with shaking of each dilution.

B) a technology-processed product resulting from serial dilutions of the stock substance of polyclonal antibodies to β2-microglobulin (β2-MG) that is obtained by combining three different successive dilutions of the stock (matrix) solution of polyclonal antibodies to β2-MG in an aqueous solvent (1.0 mg/ml). The following dilutions were subject to mixing at a volume ratio of 1:3:1:

1) a stock (matrix) solution diluted $100^{12}$ times which is equivalent to a C12 dilution, with shaking of each dilution.
2) a stock (matrix) solution diluted $100^{30}$ times which is equivalent to a C30 dilution, with shaking of each dilution.
3) a stock (matrix) solution diluted $100^{50}$ times which is equivalent to a C50 dilution, with shaking of each dilution.

C) a technology-processed product resulting from serial dilutions of the stock substance of polyclonal antibodies to interferon gamma (IFN-γ) that is obtained by combining three different successive dilutions of the stock (matrix) solution of polyclonal antibodies to IFN-γ in an aqueous solvent (2.5 mg/ml). The following dilutions were subject to mixing at a volume ratio of 1:3:1:

1) a stock (matrix) solution diluted $100^{12}$ times which is equivalent to a C12 dilution, with shaking of each dilution.
2) a stock (matrix) solution diluted $100^{30}$ times which is equivalent to a C30 dilution, with shaking of each dilution.
3) a stock (matrix) solution diluted $100^{50}$ times which is equivalent to a C50 dilution, with shaking of each dilution.

D) a technology-processed product resulting from serial dilutions of the stock substance of polyclonal antibodies to CD4 that is obtained by combining three different successive dilutions of the stock (matrix) solution of polyclonal antibodies to CD4 in an aqueous solvent (1.0 mg/ml). The following dilutions were subject to mixing at a volume ratio of 1:3:1:

1) a stock (matrix) solution diluted $100^{12}$ times which is equivalent to a C12 dilution, with shaking of each dilution.
2) a stock (matrix) solution diluted $100^{30}$ times which is equivalent to a C30 dilution, with shaking of each dilution.
3) a stock (matrix) solution diluted $100^{50}$ times which is equivalent to a C50 dilution, with shaking of each dilution.

Experimental groups:

Group 1 - animals received placebo throughout the experiment (n=30) (negative control ('viral control'));
Group 2 - mice received the comparator drug Tamiflu™ (Oseltamivir) for 5 days after infection and then open-label placebo (n=30) (positive control-comparator drug).
Group 3 - mice received the claimed medicament throughout the experiment (n=30).
Group 4 - mice received an activated-potentiated form of antibodies to interferon gamma throughout the experiment (n=30).
Group 5 - mice received an activated-potentiated form of antibodies to CD4 throughout the experiment (n=30).
Group 6 - mice received an activated-potentiated form of antibodies to HLA-DRB1 throughout the experiment (n=30).
Group 7 - mice received an activated-potentiated form of antibodies to β2-microglobulin throughout the experiment (n=30).

Study design:

Animals received the claimed medicament orally every day in the volume of 0.4 ml/mouse twice a day, in the morning and in the evening, at 10.00 a.m. and 5.00 p.m. (0.8 ml/mouse/day), for 5 days before infection, 4 hours before and 4 hours after infection and for 14 days after infection until the end of the experiment (total administration time of the test items was 20 days) ["Guidelines on non-clinical studies of pharmaceutical products" edited by A.N. Mironov (Moscow, 2012)].
The virus was inoculated into animals by intranasal route in the volume of 0.05 ml/mouse ($2.5 \times 103$ TCID50, 5 LD50) [He B, Fu Y, Xia S, et al. Intranasal application of polyethyleneimine suppresses influenza virus infection in mice. Emerging Microbes & Infections. 2016;5(4):e41-. doi:10.1038/emi.2016.64.].
The comparator drug Tamiflu™ (Oseltamivir) was administered by oral gavage at a dose of 15 mg/kg in the volume of 0.4 ml/mouse twice a day, at 10.00 a.m. and 5.00 p.m. (30 mg/mouse/day) for 5 days after infection, with the administration initiated 1 hour before infection [Oseltamivir in influenza A/H1N1 //Journal of Antimicrobial Chemotherapy, 2005, 55, p.5-21]. In this group, mice received distilled water in the volume of 0.4 ml/mouse twice a day (0.8 ml/mouse/day) for 5 days before and on Days 6-14 after infection until the end of the follow-up period. The negative control group also received placebo at a dose of 0.4 ml/mouse twice a day (0.8 ml/mouse/day) throughout the follow-up period.
All mice were examined for zootechnical parameters (live weight, survival rate, mean life expectancy (MLE), protective index), and virology testing was performed.

Study results:

The raw data on the activity of the claimed medicament showing death rates, mean life expectancy (MLE) and protective index are summarized in Table 1. The duration of the follow-up period was 14 days after infection of the animals. Mortality rates were recorded in the control and test groups on a daily basis. Based on the data obtained, the following parameters were calculated:
-mortality rate - the number of dead animals over 14 days to the total number of infected animals per group: M (%mortality) = MINt;
protection index - the difference in mortality rates (%) between in control and test groups to the mortality rate (%) in the control group: PI = ((Me - Me) / Me) $\times$ 100%;
mean animal life expectancy over 14 days of the follow-up period: MDD = ($\Sigma$ N$\times$D) / Nt; where M - the number of dead animals over 14 days per group; Me and Me - mortality % in the control (virus control) and test groups, respectively; N - the number of animals that survived D days; Nt - the total number of infected animals per group.

Table 4.

| Group | Mortality rate, % | Protection index, % | MLE, days | MLE increase, days |
|---|---|---|---|---|
| 1 (negative control) | 70 | 0.0 | 8.3 | 0 |

(continued)

| Group | Mortality rate, % | Protection index, % | MLE, days | MLE increase, days |
|---|---|---|---|---|
| 2 (Tamiflu) | 30 | 57.1 | 8.6 | 0.2 |
| 3 (Medicament) | 25 | 64.2 | 8.9 | 0.5 |
| 4 | 50 | 28.5 | 8.4 | 0.1 |
| 5 | 60 | 14.2 | 7.9 | -0.4 |
| 6 | 65 | 7.1 | 7.4 | -0.9 |
| 7 | 65 | 7.1 | 7.5 | -0.8 |

As seen from the data obtained, infection of mice with influenza A/California/097/09 (H1N1)pdm09 activated a pathological process and resulted in 75% animal mortality an average of 8.5 days after infection. The administration of the claimed medicament decreased the mortality rate to 25% (protection index 64.2%). The MLE value in this group was 8.9 days.

When analyzing body weight changes, the data from animals that were included in the mortality analysis (during 14 days after infection) were only examined.

Table 5. Body weights of experimental animals expressed as M $\pm$ SD.

| Group | Day | | | | | |
|---|---|---|---|---|---|---|
| | -15 | -5 | 0 | 5 | 9 | 12 |
| 1 (negative control) | 16.4$\pm$0.8 (N=20) | 17.9$\pm$0.9 (N=20) | 17.4$\pm$1.1 (N=20) | 15.8$\pm$1.4 (N=19) | 13.4$\pm$0.9 (N=12) | 11.9$\pm$1.1 (N=6) |
| 2 (Tamiflu) | 16.1$\pm$1.0 (N=20) | 17.8$\pm$1.2 (N=20) | 16.7$\pm$1.2 (N=20) | 15.5$\pm$1.4 (N=20) | 13.6$\pm$1.7 (N=13) | 14.9$\pm$2.2 (N=14) |
| 3 (Medicament) | 16.3$\pm$1.0 (N=20) | 17.7$\pm$1.2 (N=20) | 17.2$\pm$1.2 (N=20) | 16.1$\pm$1.3 (N=18) | 14.6$\pm$2.1 (N=15) | 15.2$\pm$2.8 (N=15) |
| 4 | 16.3$\pm$1.0 (N=20) | 17.7$\pm$1.2 (N=20) | 16.7$\pm$1.3 (N=20) | 15.2$\pm$1.4 (N=18) | 13.4$\pm$2.3 (N=9) | 14.3$\pm$2.2 (N=7) |
| 5 | 16.4$\pm$1.0 (N=20) | 17.8$\pm$1.1 (N=20) | 16.9$\pm$1.0 (N=20) | 15.3$\pm$1.1 (N=19) | 13.3$\pm$1.7 (N=11) | 13.3$\pm$2.7 (N=8) |
| 6 | 16.2$\pm$0.9 (N=20) | 18.1$\pm$1.0 (N=20) | 17.1$\pm$1.2 (N=20) | 15.8$\pm$1.1 (N=19) | 13.9$\pm$0.8 (N=8) | 12.2$\pm$1.5 (N=7) |
| 7 | 16.3$\pm$1.0 (N=20) | 17.5$\pm$0.9 (N=20) | 16.7$\pm$1.3 (N=20) | 15.4$\pm$1.1 (N=20) | 13.7$\pm$2.3 (N=12) | 12.7$\pm$2.8 (N=7) |

On Day 5 after infection of animals (n=10), viral titer was determined in the lung tissue. Mean infectious titers of influenza virus in the lungs of animals for each group are given in Table 6.

Table 6.

| Animal No. | Viral titer (lg $TCID_{50}$/0.2 ml) in the medicament group | | | | | | |
|---|---|---|---|---|---|---|---|
| | 1 | 2 | 3 | 4 | 5 | 6 | 7 |
| 1 | 5.5 | 5.5 | 4.0 | 5.5 | 5.0 | 4.5 | 5.0 |
| 2 | 6.5 | 6.0 | 5.5 | 6.5 | 6.0 | 5.5 | 5.5 |
| 3 | 6.0 | 4.5 | 2.0 | 5.0 | 5.0 | 6.0 | 6.0 |
| 4 | 6.0 | 4.5 | 1.5 | 5.0 | 4.5 | 6.0 | 5.5 |
| 5 | 6.5 | 4.5 | 5.0 | 6.0 | 5.5 | 5.5 | 6.0 |
| 6 | 6.0 | 4.5 | 4.5 | 4.5 | 6.0 | 5.0 | 5.5 |

(continued)

| Animal No. | Viral titer (lg TCID$_{50}$/0.2 ml) in the medicament group | | | | | | |
|---|---|---|---|---|---|---|---|
| | 1 | 2 | 3 | 4 | 5 | 6 | 7 |
| 7 | 6.5 | 5.0 | 5.0 | 5.0 | 6.0 | 5.5 | 5.5 |
| 8 | 5.5 | 4.5 | 4.5 | 5.5 | 4.5 | 6.0 | 5.5 |
| 9 | 6.5 | 4.0 | 3.5 | 4.5 | 5.5 | 5.5 | 5.5 |
| 10 | 7.0 | 5.0 | 4.5 | 4.5 | 5.5 | 6.5 | 6.0 |
| Mean | 6.2 | 4.8 | 3.8 | 5.2 | 5.4 | 5.6 | 5.6 |
| SD | 0.5 | 0.6 | 1.5 | 0.7 | 0.6 | 0.6 | 0.3 |
| p | 0.0562 | 0.0647 | 0.0072 | 0.4250 | 0.6743 | 0.7778 | 0.7564 |

According to the Guidelines on non-clinical studies of pharmaceutical products [Guidelines on non-clinical studies of pharmaceutical products / Ed. by A.N. Mironov. - Moscow: ZAO Grif and Co. - 2012. - Part 1. - 944 p.], a 1.75 lg and more decrease in the viral titer in a group of treated animal suggests inhibition of its replication. In this study, the claimed medicament reduced viral titer by 2.4 lgTCID$_{50}$.

Conclusion:

The highest activity in terms of mortality reduction was observed for the claimed medicament (PI=64.2), with its combined efficacy being higher than that of each component alone. Besides, the medicament of this invention exhibited pronounced antiviral effects and decreased the viral titer by 2.4 lgTCID$_{50}$.

Example 13.

Study of the antimicrobial activity of Medicaments 1 and 2 after sequential infection of animals with influenza A/New Jersey/8/76 (H1N1) virus and Streptococcus pneumoniae (S. pneumoniae) in vivo.

The aim of the study is to perform a comparative evaluation of the antimicrobial action of Medicaments 1 and 2 against pneumococcal infection caused by S. pneumoniae 7 days after infection of animals with influenza A/New Jersey/8/76 (H1N1) virus. Medicament 1 is a mixture of three aqueous-alcoholic dilutions of antibodies to human IFN-γ, antibodies to histamine and antibodies to CD4 diluted $100^{12}$, $100^{30}$, $100^{50}$ times (Ergoferon). Medicament 2 of this invention contains technology-processed products resulting from serial dilutions of the stock substances of a) antibodies to HLA-DRB1 (C12C30C50 dilution of polyclonal antibodies (2 mg/ml) at a ratio of 1:1:1), b) antibodies to β2-microglobulin (C12C30C50 dilution of polyclonal antibodies (2 mg/ml) at a ratio of 1:1:1), c) antibodies to interferon gamma (C12C30C50 dilution of monoclonal antibodies (3 mg/ml) at a ratio of 1:1:1) and d) antibodies to CD4 (C12C30C50 dilution of monoclonal antibodies (3 mg/ml) at a ratio of 1:1:1) at a volume ratio of 1:1:1:1.

Experimental groups:

Group 1 - female mice received Medicament 1 intragastrically every day at a total dose of 20 ml/kg/day (twice a day at approximately 0.2 ml/mouse at a 2 h interval) for 5 days before viral infection and 2 hours before infection on Day 0 (n=15);
Group 2 - female mice received claimed Medicament 2 intragastrically every day at a total dose of 20 ml/kg/day (twice a day at approximately 0.2 ml/mouse at a 2 h interval) for 5 days before viral infection and 2 hours before infection on Day 0 (n=15);
Group 3 - control female mice received purified water intragastrically every day at a total dose of 20 ml/kg/day (twice a day at approximately 0.2 ml/mouse at a 2 h interval) for 5 days before viral infection and 2 hours before infection on Day 0 (n=15);
Group 4 - untreated control female mice that were infected with A/New Jersey/8/76 (H1N1) and after 7 days with S. pneumoniae. This control can demonstrate placebo effect in Group 2 (n=15);
Group 5 - model control female mice that were infected S. pneumoniae alone. This control can demonstrate effects of viral pre-infection on the total CFU count (n=15).

Study design:

Animals were infected with influenza A/New Jersey/8/76 (H1N1) virus intranasally at a dose of $1 \times 10^6$ $TCID_{50}$/mouse and then infected with S. pneumoniae at $1 \times 10^6$ CFU by intratracheal route on Day 7 after viral infection. At 24, 48 and 120 hours after inoculation of S. pneumoniae, 5 mice per group were sacrificed and their lungs were isolated to evaluate the bacterial burden. The lungs collected were homogenized in a sterile phosphate buffer and serially diluted. The resultant dilutions were then plated on solid medium plates and the grown colonies were counted. The results obtained were expressed as CFU/g of lung.

Study results:

The antimicrobial activity of Medicaments 1 and 2 against pneumococcal infection caused by S. pneumoniae 7 days after infection of animals with influenza A/New Jersey/8/76 (H1N1) virus was investigated in this study. Efficacy of Medicament 1 vs. Medicament 2 in this experimental model was also compared.

Twenty-four hours after infection with S. pneumoniae, the highest CFU count for S. pneumoniae was observed in the untreated control groups (mice sequentially infected with influenza A/New Jersey/8/76 (H1N1) virus and S. pneumoniae, untreated animals) and control (mice sequentially infected with influenza A/New Jersey/8/76 (H1N1) virus and S. pneumoniae and treated with purified water) (Fig.21). By that time point, significantly decreased the lung bacterial load in virus- and bacteria-infected mice was significantly reduced by 2 and 6 times by Medicament 1 and Medicament 2 as compared to the control (Group 3), respectively, with Medicament 2 being 3 times as effective as Medicament 1.

Forty-eight hours after infection with S. pneumoniae, the highest CFU count for S. pneumoniae was still observed in the untreated control and control groups. However, absolute CFU counts in these groups showed a more than 5-fold decline. By that time point, the intensity of the antimicrobial effects of Medicament 1 was twice as higher as the control values (Groups 3 and 4), whereas the lung bacterial load in mice receiving Medicament 2 (Group 2) was 24.4% of the control value (Group 3) and 26.7% of the untreated control value (Group 4). No statistically significant differences between the Medicament 1 group and Medicament 2 group were observed.

At 120 hours after infection of mice with S. pneumoniae, isolated CFUs were only observed in all groups.

Conclusion:

The study of the antimicrobial activity of Medicaments 1 and 2 demonstrated that each of the medicaments exerted significant antibacterial effects. The comparative analysis of their specific activity showed that the claimed medicament of this invention (Medicament 2) had more pronounced effects when compared to Medicament 1.

Example 14.

Efficacy of the claimed medicament in experimental viral/bacterial pneumonia caused by influenza H1N1/A California 04/2009 virus and Staphylococcus aureus (1986 strain).

The aim of the study is to investigate the therapeutic activity of the claimed medicament in experimental viral/bacterial pneumonia caused by influenza H1N1/A California 04/2009 virus and Staphylococcus aureus (1986 strain). The medicament of this invention contains equal amounts of four technology-processed products resulting from serial dilutions (C12C30C50 dilutions) of the stock substances of affinity purified polyclonal antibodies, i.e. 1) antibodies to the β1 domain of the major histocompatibility complex class II molecule (HLA-DRB1) (2.5 mg/ml), 2) antibodies to β2-microglobulin of the major histocompatibility complex class I molecule (2.5 mg/ml), 3) antibodies to human interferon gamma (2.5 mg/ml) and 4) antibodies to CD4 (2.5 mg/ml).

Experimental groups:

Group 1 - mice received the claimed medicament intragastrically for 3 days before and 5 days after viral infection at a dose of 20 ml/kg/day (n=10);

Group 2 - mice received purified water intragastrically for 3 days before and 5 days after viral infection (20 ml/kg/day, n=10);

Group 3 - positive control 1, mice received oseltamivir (Tamiflu®, Hoffmann-La Roche, 10 mg/kg/day) by intraperitoneal route 4 hours before and 4 hours after viral infection and then twice daily during 5 days (n=10);

Group 4 - positive control 2, mice received cefuroxime (Zinacef®, GlaxoSmithKline) subcutaneously at a dose of 20 mg/kg for 3 days after bacterial infection (or 4 days after viral infection) (n=10);

Group 5 - negative control, untreated mice that were infected with influenza virus and bacteria at the similar time points as the other groups (n=10).

Study design:

Secondary viral/bacterial pneumonia was induced in animals by intranasal inoculation of influenza H1N1/A California 04/2009 virus at a dose of $10^4/5$ $TCID_{50}/0.1$ ml and intranasal inoculation of Staphylococcus aureus (1986 strain) at a dose of $2\times10^7$ CFU/ml. In all experimental groups, Staphylococcus aureus (1986) was administered on Day 5 after infection with influenza virus. Two days after, animal lungs (n=3 per group) were homogenized and examined for bacterial burden and viral titer. For the remaining mice, their body weights as a parameter of general health were measured on a daily basis as well as survival rates were recorded.

Statistical analysis:

Statistical analysis of the findings was carried out using linear mixed models including Group as a factor, Day as a covariate with a cubic spline, and Subject ID as a random effect (between-group comparisons were carried out for each day using the post-hoc Tukey test); using the Kaplan-Meier curves followed by comparison of the log-rank test with the Benjamini-Hochberg correction for multiple testing; and using the Kruskal-Wallis test in combination with the post-hoc Dunn test corrected by the Benjamini-Hochberg method. The differences were considered statistically significant if $p<0.05$.

Study results:

The claimed medicament reduced mortality rates in animals with experimental secondary (viral/bacterial) pneumonia by 40% (vs control (Group 2)), which was similar to the effect of the comparator drug cefuroxime (Group 4) (Fig.22).
The medicament of this invention, like the comparator drug oseltamivir, reduced lung viral titer by more than 15% as compared to the negative control group.
No statistically significant differences in the bacterial burden or body weights of experimental mice were observed.

Conclusion:

Thus, the claimed medicament exhibited therapeutic efficacy in experimental secondary viral/bacterial pneumonia by increasing survival rates in infected animals and reducing viral growth in the lungs.

Example 15.

Evaluation of activity against respiratory syncytial virus in vivo.
The aim of the study is to assess the antiviral activity of the claimed medicament against respiratory syncytial virus (RSV, A2 strain). The medicament of this invention contains equal amounts of four technology-processed products resulting from serial dilutions (C12C30C50 dilutions) of the stock substances of affinity purified polyclonal antibodies, i.e. 1) antibodies to the β1 domain of the major histocompatibility complex class II molecule (HLA-DRB1) (2.5 mg/ml), 2) antibodies to β2-microglobulin of the major histocompatibility complex class I molecule (2.5 mg/ml), 3) antibodies to human interferon gamma (2.5 mg/ml) and 4) antibodies to CD4 (2.5 mg/ml).

Experimental groups:

Group 1 - mice received the claimed medicament intragastrically during 5 days before infection, including 2 hours prior to infection, and then during 4 days at a dose of 20 ml/kg/day (twice a day, at approximately 0.2 ml/mouse at 2-hour intervals, n=10);
Group 2 - mice received a component of the claimed medicament (Anaferon for Children), i.e. a dilution of antibodies to interferon gamma, as monotherapy intragastrically during 5 days before infection, including 2 hours prior to infection, and then during 4 days at a dose of 20 ml/kg/day (twice a day, at approximately 0.2 ml/mouse at 2-hour intervals, n=10);
Group 3 - control mice received purified water intragastrically during 5 days before infection, including 2 hours prior to infection, and then during 4 days at a dose of 20 ml/kg/day (twice a day, at approximately 0.2 ml/mouse at 2-hour intervals, n=10);
Group 4 - RSV model control (n=10); mice were infected with RSV A2 at the same time points as mice from Group 1, Group 2 and Group 3.
Group 5 - RSV+UV model control (n=10); mice were infected with UV-inactivated RSV A2 at the same time points as mice from Group 1, Group 2 and Group 3. UV inactivation was previously performed by exposure of RSV to UV light for 20 min. An inactivated virus is an adequate negative control, since it does not cause respiratory tract infection, but virion components that are present in an inactivated viral material may still affect the parameters of the immune system of animals. This control can demonstrate the effect of the virus on the respiratory tract of animals that is achieved exactly via its replication;
Group 6 - intact control mice that were not infected with virus and remained untreated (n=10).

Study design:

Animals from five groups were infected with RSV A2 strain (RSV A2) by intraperitoneal route at a dose of $5 \times 10^6$ $TCID_{50}$/mouse. On Day 5 of the experiment, bronchial hyperresponsiveness (since RSV A2 affects the airways by causing inflammation) in animals was estimated by non-invasive plethysmography using equipment by BuxcoElectronics, Wilmington, NC. Following adjustment and calibration of the equipment, mice from different groups were placed in double chamber plethysmographs (plastic boxes) consisting of nasal and thoracic chambers divided by a neck collar as a partition to restrain animals in a plethysmograph (chamber), where the animals were challenged with 10 $\mu$l of methacholine solution of increasing concentrations 0, 6.25, 12.5 and 25 mg/ml for 3 minutes.

On Day 6 after infection, lung viral load was measured in all animals by the real-time PCR. The lung tissue collected was homogenized in an RLT buffer (Qiagen) and common RNA was isolated using the commercial RNasy mini kit (Qiagen) in compliance with the manufacturer's recommendations. Common RNA was further used as a template for the synthesis of cDNA using the OT-1 kit (Syntol) in compliance with the manufacturer's recommendations. Complementary DNA (cDNA) was used in the real-time PCR to quantify viral RNA. The real-time PCR technique was carried out using a commercial reagent kit by Syntol according to the manufacturer's recommendations, specific primer kit and IQ5 detection system (BioRad).

Statistical analysis:

Statistical analysis of the findings was carried out using the two-way ANOVA, pairwise comparisons were conducted by the least squares method, and multiplicity adjustments were performed through simulation using the SAS PROC MIXED procedure. For RNA data, multiplicity adjustments were performed using Dunnett's test (logged numbers were first calculated). The differences were considered statistically significant if $p < 0.05$.

Study results:

The highest growth of bronchial hyperresponsiveness was observed in mice of the control group that received purified water (Group 3). In mice treated with a component of the claimed medicament alone (Group 2), bronchial hyperresponsiveness demonstrated moderate growth, which was statistically different from that in the control. The lowest increase in bronchial resistance was recorded after a course of treatment with the claimed medicament (Fig.23).

The highest concentration of viral RNA was found in the specimens of mice from the RSV group (Group 4). UV inactivation of the virus resulted in a significant decline in the viral RNA count (up to 10 times), however, viral RNA could still be detected. This was due to the presence of considerable amounts of genomic RNA in UV-inactivated virus samples that was detected by the quantitative PCR. A significant difference between the viral RNA counts in the lungs of mice from the RSV (Group 4) and RSV + UV (Group 5) groups indicated that inactivated virus was capable of replication in the airways. That fact showed that the selected experimental model was adequate.

A statistically significant decrease in the viral RNA load was recorded in the specimens obtained from animals of Group 1 (the claimed medicament) and Group 2 (monotherapy with the claimed medicament component) as compared to the control group (Group 3) and RSV group (Group 4) (Fig.24). In the group of the claimed medicament, the viral RNA count was 4 times lower than that in the group of its component alone.

Conclusion:

The study of the antiviral activity of the claimed medicament on a murine model of RSV infection in vivo demonstrated that the medicament of this invention reduced bronchial hyperresponsiveness and viral RNA load in lungs. Thus, a significant antiviral action of the claimed medicament against RSV (A2 strain) was verified.

Example 16.

Efficacy of the medicament in Pseudomonas aeruginosa (RP73) infection in rats.

This is a blind, placebo-controlled, comparative study. The aim of the study is to assess the antibacterial action of the claimed medicament in experimental infection of rats with Pseudomonas aeruginosa (RP73 strain), the bacteria causing chronic lung infections with irreversible lung damage in approximately 80% of adult patients. The medicament of this invention contains four technology-processed products resulting from serial dilutions of the stock substances of affinity purifird monoclonal antibodies, i.e. 1) antibodies to HLA-DRB1 (C10D40C150 dilution (3 mg/ml) at a ratio of 2:3:1), 2) antibodies to $\beta$2-microglobulin (C10C150 dilution (3 mg/ml) at a ratio of 3:1), 3) antibodies to interferon gamma (C10D40C150 dilution (3 mg/ml) at a ratio of 2:3:1) and 4) antibodies to CD4 (C10C30C200 dilution (3 mg/ml) at a ratio of 2:3:1) at a volume ratio of 1:1:1:1.

Experimental groups:

Group 1 - male Sprague-Dawley rats (n=10) infected with Pseudomonas aeruginosa strain RP73 (by intratracheal route, $6.5*10^7$ CFU/ml) that did not receive any medicaments (untreated animals).

Group 2 - male Sprague-Dawley rats (n=10) received placebo (purified water) orally 24 hours before infection and 24, 48, 72 and 96 hours after inoculation of Pseudomonas aeruginosa strain RP73 (by intratracheal route, $6.5*10^7$ CFU/ml). Dose volume - 7.5 ml/kg.

Group 3 - male Sprague-Dawley rats (n=10) received the comparator drug tobramycin by oropharyngeal route at a dose of 3 mg/kg 24 hours before infection and 24, 48, 72 and 96 hours after inoculation of Pseudomonas aeruginosa strain RP73 (by intratracheal route, $6.5*10^7$ CFU/ml). Dose volume - 7.5 ml/kg.

Group 4 - male Sprague-Dawley rats (n=10) received the claimed test item orally 24 hours before infection and 24, 48, 72 and 96 hours after inoculation of Pseudomonas aeruginosa strain RP73 (by intratracheal route, $6.5*10^7$ CFU/ml). Dose volume - 7.5 ml/kg.

Study design:

The duration of the study was 7 days. Rats were infected with Pseudomonas aeruginosa (RP73) by intratracheal route at $6.5*10^7$ CFU/ml. Placebo (purified water) and the claimed test item were administered orally at a dose of 7.5 ml/kg 24 hours before infection and 24, 48, 72 and 96 hours after inoculation of bacteria. The comparator drug tobramycin was given to animals by oropharyngeal route at a dose of 3 mg/kg at the same time points.

Animal body weights were measured throughout the study period.

On day 5 after infection, rats were sacrificed and bronchoalveolar lavage and lung samples were collected for bacterial load analysis. The lungs were homogenized and all samples were serially diluted, and 40 $\mu$l of the resultant dilutions were then plated in triplicate on a modified Drygalski-Conradi medium for gram-negative bacteria. After 24 hours, the number of CFU was counted in the prepared samples of bronchoalveolar lavage and lungs.

Statistical analysis of the findings was carried out using the two-way repeated measures ANOVA in combination with the post-hoc Tukey test. The differences were considered statistically significant if $p < 0.05$.

Study results:

Intratracheal infection of rats with Pseudomonas aeruginosa (RP73), the most common bacteria causing chronic lung infections associated with irreversible lung damage, resulted in deterioration of the general condition of animals and associated decrease in body weights. In Group 1 (Untreated animals), this parameter declined by 27% by the end of the experiment as compared to baseline. A similar effect was also observed in Group 2 (Placebo) that exhibited a 28% decrease.

The use of the claimed medicament (Group 4) promoted improvement of the general health of infected rats: body weight loss was less pronounced compared to Groups 1 and 2, with an overall decrease being 15%. The comparator drug exerted maximum protective effects: in Group 3 (Tobramycin), the mean body weight by the end of the study only reduced by 4% (Fig. 25).

When estimating the bacterial load in the lungs and bronchoalveolar lavage samples from rats on Day 5 after intratracheal infection with Pseudomonas aeruginosa (RP73), it was found that the claimed medicament exerted significant antibacterial effects, when administered as a therapeutic and preventive course of treatment, i.e. 24 hours prior to infection and 24, 48, 72 and 96 hours after intratracheal inoculation of bacteria. As opposed to the placebo group (Group 2), where the CFU count did not show any significant differences as compared to that in Group 1 (Untreated animals), the claimed medicament reduced the lung CFU count by 29% compared to the untreated animals and by 19% compared to placebo (Fig. 26); the bronchoalveolar lavage CFU count decreased by 33% and 34%, respectively (Fig. 27). Lung bacterial load values obtained with the comparator drug were similar to those of the claimed medicament: a decrease in the lung CFU in response to tobramycin was 29% compared to the untreated animals and 19% compared to placebo. In bronchoalveolar lavage samples, the antibacterial activity of tobramycin was found to be more pronounced: 88% as compared to the two control groups (untreated animals and placebo).

Conclusion:

In the study carried out using experimental infection of rats with Pseudomonas aeruginosa (RP73 strain), the bacteria causing chronic lung infections with irreversible lung damage in approximately 80% of adult patients, the claimed medicament was shown to exert significant antibacterial effects. This was manifested by a lower weight decrease, which is a key indicator of the general health of animals, and reduced bacterial loads in the lungs (1.2-1.4-fold) and bronchoalveolar lavage samples (1.5-fold).

Example 17.

Efficacy of the medicament in mice infected with Porphyromonas gingivalis (ATCC 33277).
This is a blind placebo-controlled study. The aim of the study is to assess antibacterial and immunotropic (anti-inflammatory) effects of the claimed medicament in experimental infection of mice with Porphyromonas gingivalis, one of the main pathogens causing periodontal diseases. The medicament of this invention contains technology-processed products resulting from serial dilutions of the stock substances of affinity purified polyclonal antibodies, i.e. 1) antibodies to HLA-DRB1 (C50C200 dilution (1.5 mg/ml) at a ratio of 2:3), 2) antibodies to $\beta$2-microglobulin (C50C200 dilution (1.5 mg/ml) at a ratio of 1:2), 3) antibodies to interferon gamma (C50C200 dilution (1.5 mg/ml) at a ratio of 1:3) and 4) antibodies to CD4 (C50C200 dilution (1.5 mg/ml) at a ratio of 3:2) at a volume ratio of 2:3:2:1.

Experimental groups:

Group 1 - male C57B6/Ntac mice (n=10) infected with Porphyromonas gingivalis ATCC 33277 (subcutaneously, $10^8$ CFU/animal) that did not receive any medicaments (untreated animals).
Group 2 - male C57B6/Ntac mice (n=10) received placebo (purified water) orally 2, 24, 48 and 72 hours after infection with Porphyromonas gingivalis ATCC 33277 (subcutaneously, $10^8$ CFU/animal). Dose volume - 10 ml/kg.
Group 3 - male C57B6/Ntac mice (n=10) received the claimed medicament orally 2, 24, 48 and 72 hours after infection with Porphyromonas gingivalis ATCC 33277 (subcutaneously, $10^8$ CFU/animal). Dose volume - 10 ml/kg.

Study design:

The duration of the study was 25 days. Experimental infection was induced in mice by subcutaneous injection of Porphyromonas gingivalis (ATCC 33277) at a dose of $10^8$ CFU/animal into a pre-implanted chamber [Infect Immun. 1991 Apr;59(4):1255-63].
The test items were administered orally at a dose of 10 ml/kg 2, 24, 48 and 72 hours after infection.
On Day 4 after inoculation of bacteria, pre-implanted subcutaneous chambers were removed and the bacterial load and proinflammatory cytokine levels (IL-1$\beta$, IL-6) in the chambers were measured.
To estimate the bacterial load, 20 $\mu$l of serially diluted chamber fluids were plated in triplicate on brucella blood agar plates and incubated under anaerobic conditions for 96 hours.
Cytokine levels were measured by ELISA using test systems by R&D Systems (USA) in compliance with the manufacturer's instructions.
Statistical analysis of the findings was carried out using the Kruskal-Wallis test in combination with the post-hoc Tukey test and Kaplan-Meier curves followed by pairwise comparisons of the log-rank tests. The differences were considered statistically significant if $p < 0.05$.

Study results:

On day 4 after iv vivo injection of bacteria at $10^8$ CFU/mouse into subcutaneously implanted chambers, the bacterial load values obtained (the number of Porphyromonas gingivalis CFUs, ATCC 33277) demonstrated significant antibacterial effects of the claimed test item, when administered as a therapeutic course, i.e. 2, 24, 48 and 72 hours after infection. As opposed to the placebo group (Group 2), where the CFU count was similar to that in Group 1 (Untreated animals), the claimed medicament reduced the CFU count by 41% compared to the untreated animals and by 34% compared to placebo (Fig. 28).
The immunotropic activity of the claimed medicament was found to be significant when assessed by measuring the product's influence on the cytokine levels (IL-1β and IL-6) in subcutaneously implanted chambers on day 4 after iv vivo injection of Porphyromonas gingivalis (ATCC 33277) at a dose of $10^8$ CFU/mouse. A statistically significant decrease in the cytokine levels in response to the claimed medicament was recorded compared to the control and Group 1 (untreated animals): IL-1β declined by 49% vs Group 1 and by 41% vs Group 2 (placebo); IL-6 decreased by 60% vs Group 1 and by 63% vs Group 2 (Fig. 29, Fig. 30).

Conclusion:

Thus, in the study carried out using experimental infection of mice with Porphyromonas gingivalis, one of the main pathogens causing periodontal diseases, the claimed medicament was shown to exert significant antibacterial and immunotropic (anti-inflammatory) effects that resulted in almost 3 times lower bacterial load and decreased pro-inflammatory cytokine levels (a 1.7-fold decrease in IL-1β and a 2.7-fold decrease in IL-6) in comparison to placebo.

Example 18.

Antibacterial activity of the claimed medicament against Mycobacterium tuberculosis, H37Rv strain.
This is a blind, placebo-controlled, comparative study. The aim of the study is to assess the antibacterial action of the claimed medicament in experimental infection of mice with Mycobacterium tuberculosis (H37Rv strain). The medicament of this invention contains technology-processed products resulting from serial dilutions of the stock substances of affinity purified monoclonal antibodies, i.e. 1) antibodies to HLA-DRB1 (C12C30C50 dilution (2 mg/ml) at a ratio of 3:2:1), 2) antibodies to β2-microglobulin (C12C30C50 dilution (2 mg/ml) at a ratio of 3:2:1), 3) antibodies to interferon gamma (C12C30C50 dilution (2 mg/ml) at a ratio of 3:2:1) and 4) antibodies to CD4 (C12C30C50 dilution (2 mg/ml) at a ratio of 3:2:1) at a volume ratio of 2:2:1:2.

Experimental groups:

Group 1 - female C57BL/6 mice (n=15) infected intravenously with Mycobacterium tuberculosis, H37Rv ($5*10^4$ CFU/animal) that did not receive any medicaments (untreated animals).
Group 2 - female C57BL/6 mice (n=15) received placebo (purified water) orally during 4 weeks, from Day 22 after inoculation of Mycobacterium tuberculosis, H37Rv (by intravenous injection, $5*10^4$ CFU/animal). Dose volume - 10 ml/kg.
Group 3 - female C57BL/6 mice (n=15) received the comparator drug, i.e. isoniazid (25 mg/kg) + rifampicin (20 mg/kg) + ethambutol (100 mg/kg) combination, orally during 4 weeks, from Day 22 after inoculation of Mycobacterium tuberculosis, H37Rv (by intravenous injection, $5*10^4$ CFU/animal).
Group 4 - female C57BL/6 mice (n=15) received the claimed medicament orally during 4 weeks, from Day 22 after inoculation of Mycobacterium tuberculosis, H37Rv (by intravenous injection, $5*10^4$ CFU/animal). Dose volume - 10 ml/kg.

Study design:

The duration of the study was 50 days. Experimental infection was induced in mice by intravenous injection of Mycobacterium tuberculosis H37Rv strain at a dose of $5*10^4$ CFU/animal.
In Groups 2 and 4, the test items were administered orally at a dose of 10 ml/kg as a therapeutic course, i.e. for 28 days, from Day 22 after infection. In Group 3, the comparator drug (a combination of isoniazid (25 mg/kg) + rifampicin (20 mg/kg) + ethambutol (100 mg/kg)) was administered according to the identical regimen.
By the end of the experiment, on Day 50 after infection, mice were sacrificed and their lungs were examined for Mycobacterium tuberculosis CFU (H37Rv). The lungs isolated were homogenized and serially diluted, 20 μl of the dilutions were plated in triplicate on 7H11 agar plates and incubated for 24 hours.
Statistical analysis of the findings was carried out using the two-way ANOVA in combination with the post-hoc Tukey test. The differences were considered statistically significant if $p < 0.05$.

Study results:

The lung bacterial loads as estimated in mice infected with Mycobacterium tuberculosis (H37Rv) demonstrated a decrease in the number of CFUs in response to the claimed medicament in comparison to Group 1 (untreated animals) and Group 2 (placebo) (by 43% and 37%, accordingly). The comparator drug (a combination of isoniazid (25 mg/kg) + rifampicin (20 mg/kg) + ethambutol (100 mg/kg)) exerted similar effects by decreasing the bacterial load by 54% and 49% compared to the untreated animals and placebo groups, respectively (Fig. 31).

Conclusion:

Thus, the study carried out using a murine model of Mycobacterium tuberculosis (H37Rv) induced infection showed a significant antibacterial action of the claimed medicament that was associated with a double decrease in the lung bacterial load.

Example 19.

Evaluation of potential mutagenic activity of the claimed medicament by Salmonella typhimurium mutagenicity test.

The aim of the study is to investigate the potential mutagenicity of the claimed medicament in Salmonella typhimurium mutagenicity test (Ames test). Medicament 1 of this invention contains technology-processed products resulting from serial dilutions of the stock substances of antibodies to HLA-DRB1 (C12C30C50 aqueous dilution of polyclonal antibodies at a ratio of 1:1:1, 2,5 mg/ml) and released-active antibodies to β2-MG (C12C30C50 aqueous dilution of polyclonal antibodies at a ratio of 1:1:1, 2,5 mg/ml) at a volume ratio of 1:1. Medicament 2 of this invention contains technology-processed products resulting from serial dilutions of the stock substances of a) antibodies to HLA-DRB1 (C12C30C50 dilution of polyclonal antibodies at a ratio of 1:1:1, 2,5 mg/ml), b) antibodies to β2-microglobulin (C12C30C50 dilution of polyclonal antibodies at a ratio of 1:1:1, 2,5 mg/ml), c) antibodies to interferon gamma (C12C30C50 dilution of polyclonal antibodies at a ratio of 1:1:1, 2,5 mg/ml) and d) antibodies to CD4 (C12C30C50 dilution of polyclonal antibodies at a ratio of 1:1:1, 2,5 mg/ml) at a volume ratio of 1:1:1:1.

Study design:

The Ames test was employed using Salmonella typhimurium tester strains TA97a, TA98, TA100, TA102 and TA1535 with and without metabolic activation system. When testing in the presence of metabolic activation system, biotransformation of the test item was achieved via the use of an S9 fraction mixture containing microsomal metabolizing enzymes that was prepared by centrifugation of liver homogenate obtained from white outbred rats at 9000g. Enzyme induction was performed with sovol that was administered to rats by intraperitoneal route at a dose of 300 mg/kg.

The test medicaments and purified water (control) were tested within the assay standard dose range of 0.1-1000 μl/plate with a dilution factor of 10. The direct mutagenic activity of the claimed medicament was evaluated in assays without metabolic activation. In these assays, 100 μl of the drug and 100 μl of the bacterial culture were poured onto plates, with no microsomal mixture added to the samples. Positive controls were prepared using standard mutagens: sodium azide for TA100 strain (at a concentration of 5 μg per plate), 9-aminoacridine (10 μg/plate) for TA97 strain, 2,7-diamino-4,9-dioxy-5,10-dioxo-4,5,9,10-tetrahydro-4,9-diazopyrene (DDDTDP; 100 μg/plate) for TA98 strain. For assays without metabolic activation, the promutagen cyclophosphan was used (500 μg/plate). After the inoculation of bacteria and the test items on minimal medium plates and incubation for 2 days at 37°C, the number of histidine revertant colonies was counted. Each concentration was tested on three plates.

The geometric mean count of revertant colonies per three plates was determined for each of the experimental models. Standard deviation was calculated as the maximum deviation of the revertant count for one out of three plates from the geometric mean count. The significance of increases in the geometric mean revertant count in assays with the test composition over the control values were assessed using a modified multiple comparisons test (Dunnet's test). The differences were considered statistically significant if $p<0.05$.

Study results:

The number of revertants was similar to that in the control under the test conditions applied for the claimed medicaments.

Conclusion:

Since sovol, which enhances the activity of cytochrome P450-dependent monooxygenases of various classes, was used as a biotransformation enzyme inducer to achieve metabolic activation of the claimed medicaments, it may be concluded that possible metabolites of the medicaments of this invention do not have any mutagenic activity. Therefore, the claimed medicaments are considered to be non-mutagenic.

Example 20.

Toxicity studies of the claimed medicaments.

Toxicology studies were carried out in compliance with Good Laboratory Practice (GOST 33044-2014) and Guidelines on non-clinical studies of pharmaceutical products [Mironov AN, Bunatyan ND. Guidelines on non-clinical studies of pharmaceutical products. Moscow, Grif and Co. 2012;944 p.].

Since routine pharmacokinetic and pharmacodynamic models (dose - concentration - effect relation) approved for the majority of pharmaceutical products cannot be applied (the available physicochemical and immunological methods do not make it feasible to detect the drug concentrations in biological fluids of the organism) to the claimed medicament (a technology-processed product resulting from serial dilutions of the stock substances of antibodies to HLA-DRB1 and to β2-MG), dose selection for toxicity studies is based on physiologically acceptable volumes of a solvent (purified water in experimental studies), to which the medicament is added.

Acute toxicity:

The study was carried out on mature outbred albino mice of both sexes (body weight 22-26 g, age 2.5 months) and mature Wistar rats of both sexes (body weight 225-250 g, age 4 months) using intragastric administration. Two doses of either the test medicaments (n=18) or purified water (control, n=18) were administered intragastrically at an interval of 2 hours at the maximum dose volume: 25 ml/kg for mice and 20 ml/kg for rats. The intact group did not received any compounds (n=18). The follow-up period after administration of the items was 2 weeks.

Statistical analysis of the data obtained was carried out using the two-way ANOVA followed by between-group comparisons using the post-hoc Tukey test. The differences were considered statistically significant if $p < 0.05$.

The administration of the claimed medicament that contains a technology-processed product resulting from multiple serial dilutions of the stock substances of antibodies to HLA-DRB1 (C12C30C50 aqueous dilution of polyclonal antibodies at a ratio of 2:1:3, 2,5 mg/ml) and antibodies to β2-MG (C12C30C50 aqueous dilution of polyclonal antibodies at a ratio of 2:1:3, 2,5 mg/ml) to mature mice and rats by intragastric route at the maximum dose volumes did not affect the general health of the animals (no signs of anxiety, no changes in appetite, discharge, mucus membranes, fur, skin, etc.) or body weight gain in rats and mice (both males and females).

Similar results were obtained under the same conditions as described above using the medicament of this invention that contains technology-processed products resulting from multiple serial dilutions of the stock substances of a) antibodies to HLA-DRB1 (C12C30C50 dilution of polyclonal at a ratio of 1:3:1, 2,5 mg/ml), b) antibodies to β2-MG (C12C30C50 dilution of polyclonal at a ratio of 1:3:1, 2,5 mg/ml), c) antibodies to IFN-γ (C12C30C50 dilution of polyclonal at a ratio of 1:3:1, 2,5 mg/ml) and d) antibodies to CD4 (C12C30C50 dilution of polyclonal at a ratio of 1:3:1, 2,5 mg/ml).

The LD50 could not be calculated due to the absence of deaths among animals receiving the medicaments. Therefore, the dose exceeding the maximum dose volume of the medicament was taken as a conventional LD50.

Since two intragastric doses of the claimed medicaments at the maximum dose volumes were not found to produce any toxic effects on the health of the animals, it may be concluded that the claimed medicaments are safe and can be classified as low toxic or assigned to Category 5 as per the GHS classification.

Repeated dose toxicity:

The study was carried out on 200 conventional mature albino rats of both sexes (body weight 180-242 g, age 3.5-4 months) and 24 mature Chinchilla rabbits of both sexes (body weight 1.9 kg, age 2.5-3 months). As it was not feasible to determine the LD50 value for the medicaments, in this chronic toxicity study, the medicaments or purified water were administered to rats intragastrically at the maximum dose volume acceptable for intragastric administration and at 25% of the maximum dose volume. Rabbits received the medicament at the volume approximate to the daily water intake volume.

The period of administration was 6 months, the health of rats and rabbits was assessed 3 and 6 months after the initiation of the medicaments as well as one months after withdrawal.

A long-term intragastric administration of the medicaments of this invention to adult rats of both sexes at the doses exceeding 100 times the maximum daily dose for humans did not result in animal mortality during 6 months of administration and did not cause any significant changes in animal behavior or morphological and functional parameters of the peripheral blood as compared to the control group 3 and 6 months after the administration as well as 1 month after withdrawal of the medicament.

Moreover, a long-term administration of the medicaments at the indicated doses was not associated with any significant changes in the macro- and micromorphology and histological architecture of internal organs of rats.

In the study on rabbits, no mortality was also observed throughout the study period. A long-term administration of the medicament did not have any significant impact on the general health, behavior and body weight of animals or peripheral blood and bone marrow hematopoiesis parameters. The biochemistry of rabbit blood plasma did not reveal any significant changes. Besides, no changes in the urine of rabbits receiving the medicament for 6 months were observed. No abnormal changes were found by macroscopic examination of the internal organs of rabbits that received the medicament for 6 months. No abnormalities were observed at histological examination of organ tissues.

Conclusion:

Based on the studies conducted, it may be concluded that the claimed medicaments does not have any significant effects on the general health and behavior of animals and does not cause any abnormal or morphofunctional changes in the cardiovascular, gastrointestinal and excretory systems, when administered intragastrically to mature rats at a dose of 10 ml/kg and to rabbits at a dose of 50 ml/kg (exceeding >100 times average daily doses for human) for 6 months.

Example 21.

Multicenter, double-blind, placebo-controlled, randomized clinical trial of the claimed medicament in the treatment of acute respiratory viral infection.
The aim of the study was to assess the efficacy and safety of the claimed medicament in the treatment of acute respiratory viral infection (ARVI). The preparation according to the present invention in the form of a tablet for resorption contains, in the same amount, four technology-processed products resulting from serial dilutions (dilutions of C12C30C50) of the stock substances of polyclonal affinity purified: a) antibodies to HLA-DRB1, b) antibodies to $\beta$2-microglobulin, c) antibodies to interferon gamma and d) antibodies to CD4 applied to lactose monohydrate in the form of a water-alcohol mixture.

Study design:

The study included male and female outpatients aged 18 to 70 years (240 patients) with clinical manifestations of ARVI during the first day from the onset of the disease (axillary temperature not less than 38.0 ° C at the time of examination + total severity of general symptoms >_ 4 points, nasal / throat / chest symptoms >_2 points). The recruitment of patients was carried out during the period of seasonal incidence of ARVI.
After undergoing screening procedures, 240 patients were randomized into two groups, including 118 in the Drug group (taking the claimed medicament according to the regimen for 5 days) and 122 in the Placebo group (taking placebo according to the drug regimen for 5 days). The results of treatment and observation of these patients (n = 240) were used to assess the safety of the investigated therapy and ITT (Intention-to-treat) analysis of efficacy, including among patients with ARVI, confirmed by PCR (n = 78, Drug group and n = 73, Placebo group). Data from 6 patients (3 patients in the Drug group and 3 patients in the Placebo group) were not included in the PP (Protocol set) analysis of efficacy due to deviations from the protocol (prescription of prohibited therapy). The number of patients who received therapy in full, completed all prescribed visits and did not have significant deviations from the protocol, was 234, including 115 in the Drug group and 119 in the Placebo group. The data of these patients were used for the RR analysis of efficacy, including among patients with ARVI, confirmed by PCR (n = 76, Drug group and n = 72, Placebo group).
A patient was followed up for 14 days (screening, randomization - up to 1 day, treatment - 5 days, follow-up - up to 2 days; delayed telephone "visit" -14 days). In the course of treatment and follow-up, the patients made 3 visits to the doctor / doctor, and a fourth telephone "visit" was additionally provided. During Visits 2 and 3, the doctor conducted an objective examination, recorded the dynamics of the symptoms of the disease, concomitant therapy, and controlled the completion of the diary. At Visit 3, compliance was assessed and laboratory tests were conducted. The study also used an electronic patient diary (EPD), in which axillary body temperature and symptoms of the disease were recorded daily in the morning and in the evening.

Design of therapy:

Scheme of taking the claimed medicament: 1 tablet per dose. On the first day of treatment: the first 2 hours, 1 tablet every 30 minutes, then, during the first day, 3 more times at regular intervals. From 2 to 5 days: 1 tablet 3 times a day. The drug is taken outside of meals (in the interval between meals or 15-30 minutes before meals), keep the tablets in the mouth, without swallowing, until completely dissolved. Duration of admission is 5 days.
Comparison therapy: Placebo, lozenges, according to the regimen of the claimed drug for 5 days.
Basic and concomitant therapy: During the study, patients could receive drugs for the symptomatic therapy of ARVI: antipyretic / non-steroidal anti-inflammatory drug (paracetamol / ibuprofen) and / or vasoconstrictor nasal drug - oxymetazoline / naphazoline and / or antitussive / expectorant drug - btamyrate / ambomyrate , guaifenesin, acetylcysteine.

Samples:

Total set - these are all patients enrolled in the study who signed a patient information sheet and an informed consent form. This sample includes all adverse events (AEs) recorded during the study, including before the start of study therapy.

Safety population - all randomized patients who received at least one dose of study drug. This sample was used to analyze the safety of the investigational therapy, since all AEs identified in the patient after drug administration were recorded. AEs reported in patients in the Total set sample from the moment the informed consent was signed, but before the study drug was taken, are not considered in the safety analysis of the study therapy.

Full Analysis Set (ITT population). These are all included and randomized patients with the exception of those who have had at least one of the following events:

1) non-compliance with the inclusion / exclusion criteria;
2) the patient did not take any dose of the study drug;
3) lack of any patient data after randomization and study drug administration.

This sample, which is most consistent with the "Intention-to-treat" principle, was used for the Intention-to-treat analysis (ITT analysis) of the effectiveness of the study therapy.

To fill in missing / missing data (indicators / options), the method of transferring the data of the last observation forward "LOCF" (Last Observation Carried Forward) was used.

Per Protocol set. The sample includes all patients who received the full treatment prescribed by the protocol, completed all scheduled visits and did not have significant deviations from the protocol. This sample was used for the Per Protocol analysis (PP analysis) of the effectiveness of the study therapy.

The sample Per Protocol set did not include patients whose data were completely or partially unsuitable for analysis as a result of the revealed deviation from the protocol.

Statistical methods:

To compare the results in the two groups for continuous variables, the two-sample Student's t-test or the nonparametric Wilcoxon test was used depending on the result of the Shapiro-Wilk test for normality. Variance analysis (Mixed Procedure in SAS) was used to compare changes in indicators in the two groups. The vital signs of the patients were analyzed using the Kruskal-Wallis test. To compare the proportions (percent) in the two groups, Fisher's exact test or the Cochran-Mantel-Hensel test was used with a test for the applicability of the latter by the Breslow-Day test.

Potency assignment:

Analysis of the effectiveness of therapy by the primary endpoint

Time to the exemption of ARVI symptoms (PCR confirmed).

The time to the exemption of all symptoms of the disease in patients with ARVI, confirmed by PCR (with detected virus in nasopharyngeal samples), against the background of treatment with MMN-407 was 4.1 $\pm$ 1.9 [4.0 $\pm$ 1.9] days (versus 5.0 $\pm$ 2.5 [5.0 $\pm$ 2.5] days on the background of placebo therapy (Table 7).

Table.7. Time to the exemption of ARVI symptoms (PCR confirmed)

| | ITT analysis | | PP analysis | |
|---|---|---|---|---|
| | Drug N=78 | Placebo N=73 | Drug N=78 | Placebo N=73 |
| ARVI duration, days | | | | |
| Average $\pm$ standard deviation | 4.1 $\pm$ 1.9 | 5.0 $\pm$ 2.5 | 4.0 $\pm$ 1.9 | 5.0 $\pm$ 2.5 |
| Median | 3.5 | 4.5 | 3.5 | 4.5 |
| Lower and upper quartiles | 3.0 - 5.0 | 3.0 - 6.0 | 2.8 - 5.0 | 3.0 - 6.3 |
| 95% CI | 3.6 - 4.5 | 4.4 - 5.5 | 3.6 - 4.5 | 4.4 - 5.6 |
| $\Delta$ (between the Drug and Placebo groups) | | | | |
| Average $\pm$ standard deviation | -0.89 $\pm$ 2.23 | | -0.93 $\pm$ 2.25 | |
| 95% CI | -1.6 - -0.17 | | -1.66 - -0.20 | |

In the Drug group, the disease lasted less than 3.0 [2.8] days in 25% of patients, and in 50% - from 3.0 [2.8] to 5.0 [5.0] days (the boundaries of the lower and upper quartiles). In the placebo group, 25% of patients had symptoms of the disease for more than 6.0 [6.3] days (upper quartile border).

Among the patients of the Drug group included in the ITT analysis, the duration of the ARVI course was on average -0.89 ± 2.23 days shorter than in the Placebo group (delta between the two groups). In patients of the Drug group who completed treatment and observation in accordance with the protocol, the duration of ARVI was approximately 1 day shorter ([-0.93 ± 2.25], data from the RR analysis).

Analysis of the effectiveness of therapy by additional endpoints

1) The proportion of patients with the exemption of ARVI symptoms (clinically diagnosed and / or confirmed by PCR).

The proportion of patients with resolution of ARVI symptoms (clinically diagnosed, including PCR-confirmed ones) progressively increased starting from day 3 of treatment. In the Drug group on the 5th day of therapy, more than half of the patients (62.7 [64.4] %) were healthy (Fig. 32). At the end of 5 days of treatment, 72.9 [73.0] % of patients receiving the claimed medicament recovered from ARVI.

Statistical analysis showed that, over the entire treatment period, the percentage of patients with recovery who completed their participation in the study in accordance with the protocol was greater in the Drug group than in the Placebo group (Cochran-Mantel-Hensel test; data from the RR analysis: [p = 0.0201]).

2) Time to the exemption of ARVI symptoms (clinically diagnosed, including PCR confirmed).

Analysis of data on an additional endpoint that estimates the time to the exemption of ARVI symptoms (clinically diagnosed, including PCR confirmed among all randomized participants, also demonstrates the advantage of using the medicament in addition to symptomatic ARVI therapy (Table 8).

Table.8 Time to the exemption of ARVI symptoms (clinically diagnosed, including PCR confirmed).

| | ITT analysis | | PP analysis | |
|---|---|---|---|---|
| | Drug N=118 | Placebo N=122 | Drug N=115 | Placebo N=119 |
| ARVI duration, days | | | | |
| Average ± standard deviation | 4.4 ± 2.2 | 4.8 ± 2.4 | 4.3 ± 2.3 | 4.8 ± 2.5 |
| Median | 3.8 | 4.0 | 3.5 | 4.0 |
| Lower and upper quartiles | 3 - 5.5 | 3 - 6 | 3 - 5.5 | 3 - 6 |
| 95% CI | 4 - 4.8 | 4.3 - 5.2 | 3.9 - 4.8 | 4.4 - 5.3 |
| Δ (between the Drug and Placebo groups) | | | | |
| Average ± standard deviation | -0.41 ± 2.34 | | -0.47 ± 2.36 | |
| 95% CI | -1.00 - 0.19 | | -1.08- -0.14 | |

In the Drug group, the disease lasted less than 3.0 days in 25% of patients, and in 50% - from 3.0 to 5.5 days (the boundaries of the lower and upper quartiles). In the placebo group, 25% of patients had symptoms of the disease for more than 6.0 days (upper quartile border).

Among the patients of the Drug group included in the ITT analysis, the duration of the ARVI course, clinically diagnosed, including PCR confirmed, was on average -0.41 ± 2.34 days shorter than in the Placebo group (delta between the two groups). In patients of the Drug group who completed treatment and observation in accordance with the protocol, the duration of ARVI, clinically diagnosed, including confirmed PCR, was shorter by [-0.47 ± 2.36] days, data from the RR analysis).

Thus, the time to the exemption of the symptoms of the disease in all patients with ARVI, clinically diagnosed, including PCR-confirmed, in the Drug group was shorter than in the Placebo group..

3) The number of taking antipyretic drugs according to indications for 1-3 days of treatment.

Antipyretic medications (paracetamol or ibuprofen as dispensed by the Investigator at Visit 1) were allowed during the study, according to medical indications.

Antipyretics were prescribed on the first, second and third days of illness on average 0.30 ± 0.68 [0.28 ± 0.67], 0.19 ± 0.48 [0.20 ± 0.48] and 0.03 ± 0.22 [0.02 ± 0.13] times in the Drug group and 0.38 ± 0.67 [ 0.36 ± 0.64], 0.24 ± 0.53 [0.23 ± 0.53] and 0.04 ± 0.20 [0.04 ± 0.20] times in the Placebo group.

Statistical analysis did not reveal any differences between the groups in the number of antipyretic drugs taken within 1-3 days of observation.

Thus, the effectiveness of the claimed medicament in the treatment of patients with ARVI is shown in the absence of differences in the use of drugs for symptomatic therapy, including antipyretics.

The proportion of patients who showed a worsening of the course of the disease (the development of complications requiring antibiotics or hospitalization) from 4 to 14 days from the start of observation.

In the Drug group (N = 118), among all randomized patients with ARVI (clinically diagnosed, including PCR confirmed), acute bronchitis was diagnosed in 1 (0.8%) for which an antibacterial drug was prescribed. In the Placebo group (N = 122), 3 (2.5%) patients were prescribed antibacterial drugs due to bacterial complications, including pneumonia (n = 1 for which the patient was hospitalized), acute purulent bronchitis (n = 1), sinusitis (n = 1).

Thus, the inclusion of the claimed medicament in the ARVI treatment complex helped to prevent the worsening of the course of the disease, prevented the development of bacterial complications requiring antibacterial therapy and hospitalization of patients.

Safety Assessment:

Safety assessment and tolerability of therapy was carried out on the basis of data from all included and randomized patients who received at least one dose of the study drug / placebo (Safety population; n = 240). The vital signs of the study participants were assessed, adverse events (AEs), their relationship with the drug intake, severity, outcome were recorded; the dynamics of laboratory parameters (clinical blood analysis, general urine analysis, biochemical markers) was studied.

1) Dynamics of vital signs.

The claimed drug did not adversely affect the vital functions of the study participants, including systolic (SBP), diastolic (DBP) blood pressure, heart rate (HR) and respiratory rate (RR).

The mean SBP and DBP values throughout the study corresponded to normal and well-controlled values. Statistical analysis did not show differences in SBP, DBP, heart rate, and RR at visits 1-3 in the Drug and Placebo groups.

2) Adverse events.

In total, during the treatment and follow-up period, 9 AEs were recorded in 9 (7.6%) patients in the Drug group and 11 AEs in 9 (7.4%) participants in the Placebo group. Of all AEs, the most common were epigastric pain (n = 2, Drug group, n = 0, Placebo group), infections and invasions (including community-acquired pneumonia, n = 1, Placebo group; sinusitis, n = 1, group Placebo; acute bronchitis, n = 1, Drug group and n = 1, Placebo group; acute nasopharyntitis, n = 1, Drug group; acute tubo otitis, n = 1, Drug group; herpes simplex, n = 1, Drug group and n = 1, Placebo group), as well as general disorders and reactions at the injection site (n = 2, Placebo group in the form of lip edema, n = 1, worsening of the disease, n = 1).

In the group Drug 9 (100.0%) AEs were mild; in the Placebo group - 11 (100.0%), respectively. A causal relationship between the AE and the claimed drug under study, according to the researchers, was absent.

3) Dynamics of laboratory parameters.

In order to assess the safety of therapy, biochemical and general clinical parameters of blood and urine were studied. The average values of these laboratory parameters, both at baseline and at the end of the course of treatment, did not go beyond the reference values corresponding to normal indicators for a given age and gender.

Thus, the analysis of safety parameters showed that the medicament is safe in the treatment of patients with ARVI.

Conclusion:

The study shows that treatment with the claimed medicament for 5 days is effective and safe in patients with ARVI. The use of the medicament according to the present invention leads to a faster the exemption of ARVI symptoms (recovery), which indicates the advantages of including the drug in the ARVI therapy complex.

**Claims**

1. A medicament, which presents a technology-processed product resulting from multiple serial dilutions of the stock substances of antibodies to the $\beta1$ domain of the major histocompatibility complex class II molecule (HLA-DRB1) and to $\beta2$-microglobulin ($\beta2$-MG); wherein the multiple serial dilutions are decimal, centesimal and millesimal dilutions in any dilution ratios; and wherein the stock substances are at a concentration of 0.5 ÷ 5.0 mg/ml.

2. The medicament of claim 1 for the treatment of bacterial infections; optionally wherein the bacterial infection is (i) caused by bacteria resistant to one or more known antibiotics; (ii) a urinary tract infection selected from cystitis and prostatitis; (iii) is a bacterial intestinal infection; (iv) a bacterial skin infection; (v) tuberculosis; or (vii) selected from salmonellosis, E.coli infection, streptococcal infection and Klebsiella pneumoniae.

3. The medicament of claim 1, wherein the antibodies are monoclonal, polyclonal or natural.

4. The medicament of claim 1 formulated as a solid dosage form, which contains effective amounts of neutral carrier granules impregnated with a technology-processed product resulting from multiple serial dilutions of the stock substances of antibodies to HLA DRB1 and antibodies to $\beta2$-microglobulin as well as pharmaceutically acceptable excipients.

5. A combination of an antibiotic and the medicament of any of claims 1 to 4, for either simultaneous or successive administration to treat bacterial infections; optionally wherein the bacterial infection is (i) caused by bacteria resistant to one or more known antibiotics; (ii) a urinary tract infection selected from cystitis and prostatitis; (iii) is a bacterial intestinal infection; (iv) a bacterial skin infection; (v) tuberculosis; or (vii) selected from salmonellosis, E.coli infection, streptococcal infection and Klebsiella pneumoniae.

6. An antibiotic and the medicament of claim 1 for use in a method of reducing bacterial resistance to antibiotic therapy, which comprises either simultaneous or successive co-administration of the antibiotic and the medicament of claim 1.

7. The antibiotic and medicament for use according to claim 6, wherein the antibiotic is administered at the known effective dose.

8. The medicament of claim 1 for use in a method of enhancing antibiotic efficacy, which comprises either simultaneously or successively administering the medicament of claim 1.

9. The combination of claim 5, the antibiotic and medicament for use of claim 6 or the medicament for use of claim 8; wherein the antibiotic is selected from the class of penicillins, aminoglycosides, macrolides and oxazolidinones.

10. The medicament for use of claim 8, wherein the antibiotic is administered at 50% of the effective dose (ED50).

11. A medicament, which presents a technology-processed product resulting from multiple serial dilutions of the stock substances of a) antibodies to the $\beta1$ domain of the major histocompatibility complex class II molecule (HLA-DRB1), b) antibodies to $\beta2$-microglobulin $\beta2$-MG), c) antibodies to interferon gamma (IFN-$\gamma$) and d) antibodies to CD4; wherein the multiple serial dilutions are decimal, centesimal and millesimal dilutions in any dilution ratios; and wherein the stock substances are at a concentration of 0.5 ÷ 5.0 mg/ml.

12. The medicament of claim 11 for the treatment of infectious diseases; optionally wherein the infectious disease is (i) a viral infection; optionally selected from URI, influenza; (ii) a bacterial infection; (iii) a mixed infection (iv) a secondary infection; optionally wherein the secondary infection is viral/bacterial pneumonia.

13. The medicament of claim 1 or claim 11, wherein the technology-processed product resulting from multiple serial dilutions of the stock substance of antibodies is an aqueous or aqueous-alcoholic solution obtained from serial dilutions of the matrix solution of antibodies, with repeated shaking of each dilution; optionally wherein the matrix solution of antibodies is used at a concentration of 0.5 ÷ 5.0 mg/ml.

14. The medicament of any of claims 11 to 13, wherein the antibodies are monoclonal, polyclonal or natural.

15. The medicament of claim 11 formulated as a solid dosage form, which contains effective amounts of neutral carrier granules impregnated with a technology-processed product resulting from multiple serial dilutions of the stock substances of antibodies to HLA DRB1, antibodies to $\beta2$-MG, antibodies to IFN-$\gamma$ and antibodies to CD4 as well as pharmaceutically acceptable excipients.

**Patentansprüche**

1.  Medikament, das ein technisch verarbeitetes Produkt darstellt, das durch mehrere serielle Verdünnungen der Stammsubstanzen von Antikörpern gegen die β1-Domäne des Haupthistokompatibilitäts-Komplex-Klasse-II-Moleküls (HLA-DRB1) und gegen β2-Mikroglobulin (β2-MG) gewonnen wird; wobei es sich bei den mehreren seriellen Verdünnungen um dezimale, zentesimale und millesimale Verdünnungen mit beliebigen Verdünnungsverhältnissen handelt und wobei die Stammsubstanzen mit einer Konzentration von 0,5 - 5,0 mg/ml vorliegen.

2.  Medikament nach Anspruch 1 zur Behandlung von bakteriellen Infektionen; wobei die bakterielle Infektion gegebenenfalls (i) von Bakterien, die gegenüber einem oder mehreren bekannten Antibiotika resistent sind; (ii) einer Harnwegsinfektion verursacht wird, die aus Zystitis und Prostatitis ausgewählt ist; (iii) eine bakterielle Darminfektion; (iv) eine bakterielle Hautinfektion; (v) Tuberkulose ist oder (vii) aus einer Salmonellose, E.-coli-Infektion, Streptokokkeninfektion und Klebsiella pneumoniae ausgewählt ist.

3.  Medikament nach Anspruch 1, wobei die Antikörper monoklonal, polyklonal oder natürlich sind.

4.  Medikament nach Anspruch 1, das als feste Dosierungsform formuliert ist, die wirksame Mengen eines neutralen Trägergranulats, das mit einem technisch verarbeiteten Produkt getränkt ist, das durch mehrere serielle Verdünnungen der Stammsubstanzen von Antikörpern gegen HLA-DRB1 und Antikörpern gegen β2-Mikroglobulin gewonnen wird, sowie pharmazeutisch annehmbare Hilfsstoffe enthält.

5.  Kombination aus einem Antibiotikum und dem Medikament nach einem der Ansprüche 1 bis 4 zur entweder gleichzeitigen oder aufeinanderfolgenden Verabreichung zur Behandlung von bakteriellen Infektionen; wobei die bakterielle Infektion gegebenenfalls (i) von Bakterien, die gegenüber einem oder mehreren bekannten Antibiotika resistent sind; (ii) einer Harnwegsinfektion verursacht wird, die aus Zystitis und Prostatitis ausgewählt ist; (iii) eine bakterielle Darminfektion; (iv) eine bakterielle Hautinfektion; (v) Tuberkulose ist oder (vii) aus einer Salmonellose, E.-coli-Infektion, Streptokokkeninfektion und Klebsiella pneumoniae ausgewählt ist.

6.  Antibiotikum und Medikament nach Anspruch 1 zur Verwendung bei einem Verfahren zur Reduzierung einer Bakterienresistenz gegenüber einer Antibiotikatherapie, die entweder die gleichzeitige oder die aufeinanderfolgende gemeinsame Verabreichung des Antibiotikums und des Medikaments nach Anspruch 1 umfasst.

7.  Antibiotikum und Medikament zur Verwendung nach Anspruch 6, wobei das Antibiotikum mit der bekannten wirksamen Dosis verabreicht wird.

8.  Medikament nach Anspruch 1 zur Verwendung bei einem Verfahren zur Verstärkung der antibiotischen Wirksamkeit, das die entweder gleichzeitige oder aufeinanderfolgende Verabreichung des Medikaments nach Anspruch 1 umfasst.

9.  Kombination nach Anspruch 5, Antibiotikum und Medikament zur Verwendung nach Anspruch 6 oder Medikament zur Verwendung nach Anspruch 8; wobei das Antibiotikum aus der Klasse von Penicillinen, Aminoglycosiden, Makroliden und Oxazolidinonen ausgewählt ist.

10. Medikament zur Verwendung nach Anspruch 8, wobei das Antibiotikum mit 50 % der wirksamen Dosis (ED50) verabreicht wird.

11. Medikament, das ein technisch verarbeitetes Produkt darstellt, das durch mehrere serielle Verdünnungen der Stammsubstanzen von a) Antikörpern gegen die β1-Domäne des Haupthistokompatibilitäts-Komplex-Klasse-II-Moleküls (HLA-DRB1), b) Antikörper gegen β2-Mikroglobulin (β2-MG), c) Antikörper gegen Interferon-Gamma (IFN-γ) und d) Antikörper gegen Cd4 gewonnen wird; wobei es sich bei den mehreren seriellen Verdünnungen um dezimale, zentesimale und millesimale Verdünnungen mit beliebigen Verdünnungsverhältnissen handelt und wobei die Stammsubstanzen mit einer Konzentration von 0,5 - 5,0 mg/ml vorliegen.

12. Medikament nach Anspruch 11 zur Behandlung von Infektionskrankheiten; wobei es sich bei der Infektionskrankheit gegebenenfalls um (i) eine Virusinfektion, die gegebenenfalls aus einer URI, Influenza, ausgewählt ist; (ii) eine bakterielle Infektion; (iii) eine gemischte Infektion, (iv) eine sekundäre Infektion handelt; wobei es sich bei der sekundären Infektion gegebenenfalls um eine virale/bakterielle Pneumonie handelt.

13. Medikament nach Anspruch 1 oder Anspruch 11, wobei das technisch verarbeitete Produkt, das durch mehrere serielle Verdünnungen der Stammsubstanz von Antikörpern gewonnen wird, eine wässrige oder wässrig-alkoholische Lösung ist, die durch mehrere serielle Verdünnungen der Matrixlösung von Antikörpern mit wiederholtem Schütteln einer jeden Verdünnung gewonnen wird; wobei die Matrixlösung von Antikörpern gegebenenfalls mit einer Konzentration von 0,5 - 5,0 mg/ml verwendet wird.

14. Medikament nach einem der Ansprüche 11 bis 13, wobei die Antikörper monoklonal, polyklonal oder natürlich sind.

15. Medikament nach Anspruch 11, das als feste Dosierungsform formuliert ist, die wirksame Mengen eines neutralen Trägergranulats, das mit einem technisch verarbeiteten Produkt getränkt ist, das durch mehrere serielle Verdünnungen der Stammsubstanzen von Antikörpern gegen HLA-DRB1, Antikörpern gegen β2-MG, Antikörpern gegen IFN-γ und Antikörpern gegen CD4 gewonnen wird, sowie pharmazeutisch annehmbare Hilfsstoffe enthält.

**Revendications**

1. Médicament, qui présente un produit traité par technologie résultant de dilutions en série multiples des substances mères d'anticorps contre le domaine pi de la molécule du complexe majeur d'histocompatibilité de classe II (HLA-DRB1) et contre la β2-microglobuline (β2-MG) ; dans lequel les dilutions multiples en série sont des dilutions décimales, centésimales et millésimales dans tous les rapports de dilution ; et dans lequel les substances mères sont à une concentration de 0,5 ÷ 5,0 mg/ml.

2. Médicament selon la revendication 1 pour le traitement d'infections bactériennes ; éventuellement dans lequel l'infection bactérienne est (i) causée par des bactéries résistantes à un ou plusieurs antibiotiques connus ; (ii) une infection des voies urinaires sélectionnée parmi la cystite et la prostatite ; (iii) est une infection bactérienne intestinale ; (iv) une infection bactérienne de la peau ; (v) la tuberculose ; ou (vii) choisie parmi une salmonellose, une infection par E. coli, une infection par des streptocoques et Klebsiella pneumoniae.

3. Médicament selon la revendication 1, dans lequel les anticorps sont monoclonaux, polyclonaux ou naturels.

4. Médicament selon la revendication 1 formulé sous une forme posologique solide, qui contient des quantités efficaces de granules porteurs neutres imprégnés d'un produit traité par technologie résultant de dilutions en série multiples des substances mères d'anticorps contre HLA DRB1 et des anticorps contre la β2-microglobuline ainsi que des excipients pharmaceutiquement acceptables.

5. Combinaison d'un antibiotique et du médicament selon l'une quelconque des revendications 1 à 4, pour une administration soit simultanée, soit successive afin de traiter des infections bactériennes ; éventuellement, l'infection bactérienne étant (i) causée par des bactéries résistantes à un ou plusieurs antibiotiques connus ; (ii) une infection des voies urinaires sélectionnée parmi la cystite et la prostatite ; (iii) une infection bactérienne intestinale ; (iv) une infection bactérienne de la peau ; (v) la tuberculose ; ou (vii) une infection sélectionnée parmi la salmonellose, une infection par E. coli, une infection par des streptocoques et Klebsiella pneumoniae.

6. Antibiotique et médicament selon la revendication 1 pour une utilisation dans un procédé de réduction de la résistance bactérienne à une thérapie par antibiotiques, qui comprend une administration soit simultanée, soit successive de l'antibiotique et du médicament selon la revendication 1.

7. Antibiotique et médicament pour une utilisation selon la revendication 6, l'antibiotique étant administré à la dose efficace connue.

8. Médicament selon la revendication 1 pour utilisation dans un procédé d'amélioration de l'efficacité antibiotique, qui comprend une administration soit simultanée, soit successive du médicament selon la revendication 1.

9. Combinaison selon la revendication 5, antibiotique et médicament pour une utilisation selon la revendication 6 ou médicament pour une utilisation selon la revendication 8 ; l'antibiotique étant sélectionné dans la classe des pénicillines, des aminoglycosides, des macrolides et des oxazolidinones.

10. Médicament pour une utilisation selon la revendication 8, l'antibiotique étant administré à 50 % de la dose efficace (ED50).

**11.** Médicament, qui présente un produit traité par technologie résultant de dilutions multiples en série des substances mères de a) des anticorps contre le domaine β1 de la molécule du complexe majeur d'histocompatibilité de classe II (HLA-DRB1), b) des anticorps contre la β2-microglobuline (β2-MG), c) des anticorps contre l'interféron gamma (IFN-γ) et d) des anticorps contre CD4 ; les dilutions en série multiples étant des dilutions décimales, centésimales et millésimales dans tous les rapports de dilution ; et les substances mères étant à une concentration de 0,5 ÷ 5,0 mg/ml.

**12.** Médicament selon la revendication 11 pour le traitement de maladies infectieuses ; éventuellement la maladie infectieuse étant (i) une infection virale ; éventuellement sélectionnée parmi l'URI, la grippe ; (ii) une infection bactérienne ; (iii) une infection mixte (iv) une infection secondaire ; éventuellement, l'infection secondaire étant une pneumonie virale/bactérienne.

**13.** Médicament selon la revendication 1 ou la revendication 11, le produit traité par technologie résultant de dilutions multiples en série de la substance mère d'anticorps étant une solution aqueuse ou aqueuse alcoolique obtenue à partir de dilutions en série de la solution matricielle d'anticorps, avec agitation répétée de chaque dilution ; éventuellement, la solution matricielle d'anticorps étant utilisée à une concentration de 0,5 ÷ 5,0 mg / ml.

**14.** Médicament selon l'une quelconque des revendications 11 à 13, les anticorps étant monoclonaux, polyclonaux ou naturels.

**15.** Médicament selon la revendication 11 formulé sous forme posologique solide, qui contient des quantités efficaces de granules porteurs neutres imprégnés d'un produit traité par technologie résultant de dilutions en série multiples des substances mères d'anticorps contre HLA DRB1, d'anticorps contre β2-MG, d'anticorps contre IFN-γ et d'anticorps contre CD4 ainsi que des excipients pharmaceutiquement acceptables.

Bladder weight, mg

Fig. 1.

Bacterial load, CFU/g

Fig. 2.

Kidney weight, mg

Fig. 3.

Bacterial load, CFU/g

Fig. 4.

Fig. 5.

Fig. 6.

Fig. 7.

Fig. 8.

AMC IC50, $\mu$g/ml

% bacterial growth inhibition
AMC at a dose of 1080, $\mu$g/ml

Fig. 9.

Fig. 10

Fig. 11.

Fig. 12.

Fig. 13.

Fig. 14.

Fig. 15.

Fig. 16

Fig. 17.

Fig. 18.

Fig. 19.

Fig. 20.

Fig. 21.

Fig. 22.

Fig. 23.

Fig. 24.

Fig. 25.

Fig. 26.

Fig. 27.

Fig. 28.

Fig. 29.

Fig. 30.

Fig. 31.

Fig. 32.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2363470 C1
- RU 2655808 C2
- RU 2205025 C1
- RU 2521392
- RU 2500422 C2
- WO 2007105981 A1

**Non-patent literature cited in the description**

- **LENEVA I.A. et al.** Characteristics of arbidol-resistant mutants of influenza virus: implications for the mechanism of anti-influenza action of arbidol. *Antiviral Res,* 2009, vol. 81 (2), 132-40
- **BELOZERTSEVA V.N. et al.** Antibiotics: resistance and toxicity. *New St. Petersburg Medical Records,* 2017, vol. 2 (2), 59-61
- **I.V. SMIRNOV.** Pathogens of bacterial infections in humans. *Clinical Microbiology and Antimicrobial Chemotherapy,* 2000, vol. 2 (2), 4-11
- **EPSTEIN O.I.** Released-activity (contemporary view on homeopathy and non-homeopathy). Moscow. RAMS Publishing House, 2017, 48
- Epstein O.I. Ultralow doses (history of one study). RAMS Publishing House, 2008, 336
- **MYAGKOVA M.A. ; MOROZOVA V.S.** Immunochemical properties of natural antibodies to physiologically active compounds. *Fundamental Research,* 2014, vol. 11 (11), 1066-1070
- Immunological methods. Meditsina, 1987, 9-33
- **LAFFLY E. ; SODOYER R.** Monoclonal and recombinant antibodies, 30 years after. *Hum. Antibodies,* 2005, vol. 14 (1-2), 33-55
- **LIPMAN NS1 ; JACKSON LR ; TRUDEL LJ ; WEIS-GARCIA F.** Monoclonal versus polyclonal antibodies: distinguishing characteristics, applications, and information resources. *ILAR J,* 2005, vol. 46 (3), 258-68
- IHC Staining Methods. Antibodies. Thomas Boenisch, vol. 1, 1-9
- **LIPMAN NS1 ; JACKSON LR ; TRUDEL LJ ; WEIS-GARCIA F.** Monoclonal versus polyclonal antibodies: distinguishing characteristics, applications, and information resources. *ILAR J,* 2005, vol. 46 (3), 258-68
- **BOYD WC.** Fundamentals of immunology. *Fundam. Immunol,* 1946
- **LANGMAN RE.** The specificity of immunological reactions. *Mol. Immunol,* 2000, vol. 37, 555-561
- **A. ROITT et al.** Essential Immunology. *Moscow: Mir,* 2000, 149-161, 527-544
- **W. SHWABE.** *Homeopathic medicines,* 1967, 14-29
- **OKHAPKINA V.Y. ; PYATKOVA N.V. ; FEDOTOV A.K.** Express efficacy assessment method for antibacterial agents in experimental brucellosis. *Challenges of Highly Infectious Diseases,* 2016, vol. 2, 79-82
- Guidelines on non-clinical studies of pharmaceutical products. 2012
- **HE B ; FU Y ; XIA S et al.** Intranasal application of polyethyleneimine suppresses influenza virus infection in mice. *Emerging Microbes & Infections,* 2016, vol. 5 (4), e41
- *Journal of Antimicrobial Chemotherapy,* 2005, vol. 55, 5-21
- Guidelines on non-clinical studies of pharmaceutical products. ZAO Grif and Co, 2012, vol. 1, 944
- *Infect Immun,* April 1991, vol. 59 (4), 1255-63
- **MIRONOV AN ; BUNATYAN ND.** Guidelines on non-clinical studies of pharmaceutical products. Moscow. Grif and Co, 2012, 944